# EUROPEAN PATENT APPLICATION

(11) **EP 4 538 379 A1**
(43) Date of publication of application: **16.04.2025**
(21) Application number: 23819929.3
(22) Date of filing: 09.06.2023
(51) Int. Cl.: C12N 15/85, C07K 16/00, C07K 16/46, C12N 5/10, C12N 15/13, C12N 15/62, C12P 21/02

(54) **METHOD FOR PRODUCING CELLS, METHOD FOR PRODUCING HETEROMULTIMERIC PROTEIN, METHOD FOR PRODUCING BISPECIFIC ANTIBODY, AND METHOD FOR PRODUCING VECTOR SET, MAMMALIAN CELLS, CHO CELLS, AND CELL POOL**

(30) Priority: 10.06.2022 JP 2022094577
(71) Applicant: Fujifilm Diosynth Biotechnologies UK Limited, Billingham TS23 1LH (GB)
(72) Inventor: MATSUURA, Tatsuya, Ashigarakami-gun, Kanagawa 258-8577 (JP); KURODA, Akari, Ashigarakami-gun, Kanagawa 258-8577 (JP)
(74) Representative: HGF
(86) International application number: PCT/JP2023/021621
(87) International publication number: WO 2023/238949

(57) **Abstract**

This vector set is induced into host cells, and cells for producing heteromultimeric proteins are selected from the host cells into which has the vector set is introduced. The vector set is a set of a first expression vector and a second expression vector, wherein an expression cassette of at least one subunit X which is a part of a subunit constituting a heteromultimeric protein is contained in both the first expression vector and the second expression vector and from among the subunits constituting the heteromultimeric protein, an expression cassette of the remaining subunit Y excluding the subunit X is contained for each type in one among the first expression vector and the second expression vector.

## Description

### Technical Field

The present disclosure relates to a method for producing cells, a method for producing heteromultimeric proteins, a method for producing bispecific antibodies, and a method for producing vector sets, mammalian cells, CHO cells, and cell pools.

### Background Art

Patent Literature 1 discloses a method of selecting cells expressing a bispecific antibody including transducing eukaryotic cells with lentiviruses.

Patent Literature 2 discloses production of bispecific anti-HER2 antibodies by culturing host cells containing an expression vector for the bispecific anti-HER2 antibodies.

Patent Literature 3 discloses multispecific antigen-binding molecules that include (1) a domain containing an antibody variable region having glypican 3 binding activity, (2) a domain containing an antibody variable region having T cell receptor complex binding activity, and (3) a domain containing an Fc region with reduced Fcy receptor binding activity, wherein an L-chain variable region contained in the variable region of (1) and the variable region of (2) is a common amino acid sequence.

Patent Literature 4 discloses a multispecific antigen-binding molecule that contains a first antigen binding site recognizing blood coagulation factor IX and a second antigen binding site recognizing blood coagulation factor X, and that has a function substituting for a function of blood coagulation factor VIII.

Patent Literature 5 discloses a bispecific antibody containing an antigen binding domain that binds to CD40 and an antigen binding domain that binds to EpCAM.

Patent Literature 6 discloses a bispecific anti-HER2 antibody having a common light chain and two different heavy chains.

Patent Literature 7 discloses a bispecific antibody that binds to the extracellular portion of PD-1 and the extracellular portion of TIM-3.

Patent Literature 8 discloses a bispecific antibody that binds to hPD-L1, and TIGIT or LAG-3.

Patent Literature 9 discloses a bispecific antibody that binds to CD38 and PD-L1.

Patent Literature 10 discloses fibronectin secretion leaders that facilitate the secretion of a target polypeptide to be produced in host cells.

### Citation List

### Patent Literature

Patent Literature 1: Japanese Translation of PCT International Application Publication No. 2015-503907
Patent Literature 2: Japanese Translation of PCT International Application Publication No. 2017-501706
Patent Literature 3: International Publication No. WO 2016/047722
Patent Literature 4: International Publication No. WO 2012/067176
Patent Literature 5: International Publication No. WO 2019/093342
Patent Literature 6: Japanese Translation of PCT International Application Publication No. 2018-504113
Patent Literature 7: Japanese Translation of PCT International Application Publication No. 2020-532281
Patent Literature 8: Japanese Translation of PCT International Application Publication No. 2019-528083
Patent Literature 9: Japanese Translation of PCT International Application Publication No. 2019-516396
Patent Literature 10: International Publication No. WO 2014/177826

### Summary of Invention

### Technical Problem

Conventionally, expression vectors for subunits constituting heteromultimeric proteins have been introduced into host cells for the purpose of producing cells that produce heteromultimeric proteins. For example, when a heteromultimeric protein consists of one subunit A, one subunit B, and one subunit C, an expression vector containing each one of the expression cassette of the subunit A, the expression cassette of the subunit B, and the expression cassette of the subunit C is introduced into host cells. When simply predicting, the above three subunits will be expressed at an expression level ratio of 1:1:1, and a heteromultimeric protein will be produced. In reality, however, the expression level or purity of the heteromultimeric protein may be low, and the amount of the heteromultimeric protein produced may not reach the expected value. Possible reasons for this may include the fact that each subunit has a different transcription rate or translation rate; some subunits are easily degraded; and all subunits are aggregated when a certain type of subunit is in excess number.

As a means of increasing the expression level or purity of the heteromultimeric protein, it is conceivable that the optimal ratio of the expression levels of the subunits is predetermined, and at a number ratio according to the ratio thus obtained, the expression cassettes of the subunits are placed on one expression vector. However, it is not always easy to know the optimal ratio of the expression levels of subunits.
Also, when the optimal ratio of the expression levels of subunits is a relatively large integer (e.g., 1:2:3), the total number of expression cassettes to be placed on one expression vector increases. As a result, the size of the expression vector increases, and the introduction rate into host cells decreases.

As another means of increasing the expression level or purity of the heteromultimeric protein, it is conceivable that each expression cassette of the subunit is placed on a distinct expression vector, and multiple types of expression vectors are introduced into host cells. For example, when a heteromultimeric protein consists of a subunit A, a subunit B, and a subunit C, an expression vector A for the subunit A, an expression vector B for the subunit B, and an expression vector C for the subunit C are introduced into host cells. The numbers of the expression vector A, expression vector B, and expression vector C to be introduced into host cells vary depending on the host cells, and hence, diversity occurs in the number of each expression cassette possessed by the host cells. Cells with a high expression level or purity of the heteromultimeric protein are selected from a variety of host cells. However, it is required to construct the same number of types of expression vectors as the number of types of subunits constituting the heteromultimeric protein, and to prepare the same number of types of selection markers as the number of types of subunits.

The present disclosure provides a means different from the above two means, as the means of increasing the expression level or purity of the heteromultimeric protein.

An object of an embodiment of the present disclosure is to provide a method for producing cells excellent in productivity of a heteromultimeric protein or a bispecific antibody.

An object of an embodiment of the present disclosure is to provide a method for producing a heteromultimeric protein or a bispecific antibody excellent in productivity.

An object of an embodiment of the present disclosure is to provide a vector set used for producing cells that produce a heteromultimeric protein or a bispecific antibody.

An object of an embodiment of the present disclosure is to provide mammalian cells and CHO cells that produce a heteromultimeric protein or a bispecific antibody.

An object of an embodiment of the present disclosure is to provide a method for producing a cell pool used for selecting cells that produce a heteromultimeric protein or a bispecific antibody.

### Solution to Problem

A method for producing cells of the present disclosure includes introducing a first expression vector and a second expression vector, in other words, two types of expression vectors into a host cell in order to express a heteromultimeric protein.

An expression cassette of a subunit of interest (usually, a subunit whose expression is desired to be enhanced) is placed on both the first expression vector and the second expression vector. The subunit of interest may be in one type, or may be in two or more. Expression cassettes of the rest of the subunits other than the subunit of interest are placed on one of the first expression vector and the second expression vector for each type.

The expression of the entire subunits is ensured by introducing both the first expression vector and the second expression vector into the host cell. Since the expression cassette of the subunit of interest is present on both the first expression vector and the second expression vector, the amount introduced is relatively increased, and the expression of the subunit of interest can be enhanced.

The numbers of the first expression vector and the second expression vector to be introduced into the host cell vary depending on the host cell, and hence, diversity occurs in the number of each expression cassette possessed by the host cell. Cells that satisfy evaluation criteria (e.g., the expression level or purity of the heteromultimeric protein) are selected from a variety of host cells.

Specific means to solve the problem include the following aspects.

<1> A method for producing cells that produce a heteromultimeric protein consisting of n types of subunits (wherein, n is an integer of 2 or higher), including:
   introducing a vector set into a host cell; and
   selecting cells that produce a heteromultimeric protein from the host cell into which the vector set has been introduced,
   in which
   the vector set is a set of a first expression vector and a second expression vector,
   an expression cassette of at least one subunit X that is a portion of the n types of subunits is contained in both the first expression vector and the second expression vector, and
   expression cassettes of subunits Y remaining after the subunit X is removed from the n types of subunits are contained in one of the first expression vector and the second expression vector for each type.
<2> The method for producing cells according to <1>, in which the vector set is a vector set below,
   in which
   the vector set is the set of the first expression vector and the second expression vector,
   the expression cassette of at least one subunit X that is a portion of the n types of subunits is contained in both the first expression vector and the second expression vector, and
   all of the expression cassettes of the subunits Y remaining after the subunit X is removed from the n types of subunits are contained in one of the first expression vector and the second expression vector.
<3> The method for producing cells according to <1> or <2>, in which
   the heteromultimeric protein consisting of the n types of subunits is a heteromultimeric protein consisting of two or more types and six or fewer types of subunits, and
   the at least one subunit X is one type, two types, or three types of subunits X.
<4> The method for producing cells according to <1> or <2>, in which
   the heteromultimeric protein consisting of the n types of subunits is a heteromultimeric protein consisting of three or four types of subunits, and
   the at least one subunit X is one type, two types, or three types of subunits X.
<5> The method for producing cells according to <1> or <2>, in which
   the heteromultimeric protein consisting of the n types of subunits is a heteromultimeric protein consisting of three types of subunits, and
   the at least one subunit X is one type or two types of subunits X.
<6> The method for producing cells according to any one of <1> to <5>, in which
   the heteromultimeric protein is an antibody.
<7> The method for producing cells according to any one of <1> to <5>, in which
   the heteromultimeric protein is a bispecific antibody.
<8> The method for producing cells according to any one of <1> to <7>, in which
   all of the expression cassettes of the subunits constituting the heteromultimeric protein contain promoters of the same type.
<9> The method for producing cells according to <8>, in which
   the promoter is an hEF-1α promoter.
<10> The method for producing cells according to any one of <1> to <9>, in which
   at least one of the expression cassettes of the subunits constituting the heteromultimeric protein contains a coding sequence of a fibronectin secretion leader, and
   in the expression cassettes containing the coding sequence of the fibronectin secretion leader, a coding sequence of the subunits constituting the heteromultimeric protein is arranged downstream of the coding sequence of the fibronectin secretion leader in the same reading frame.
<11> The method for producing cells according to any one of <1> to <9>, in which
   all of the expression cassettes of the subunits constituting the heteromultimeric protein contain a coding sequence of a fibronectin secretion leader, and
   a coding sequence of the subunits constituting the heteromultimeric protein is arranged downstream of the coding sequence of the fibronectin secretion leader in the same reading frame.
<12> The method for producing cells according to any one of <1> to <11>, in which
   the first expression vector further contains an expression cassette of a first selection marker, and
   the second expression vector further contains an expression cassette of a second selection marker.
<13> The method for producing cells according to <12>, in which
   the first selection marker and the second selection marker are selection markers different from each other,
   the first selection marker is at least one selected from the group consisting of a dihydrofolate reductase gene, a glutamine synthetase gene, and an antibiotic resistance gene, and
   the second selection marker is at least one selected from the group consisting of a dihydrofolate reductase gene, a glutamine synthetase gene, and an antibiotic resistance gene.
<14> The method for producing cells according to any one of <1> to <13>, in which the host cell is a mammalian cell.
<15> The method for producing cells according to any one of <1> to <13>, in which the host cell is a Chinese hamster ovary cell (CHO cell).
<16> The method for producing cells according to any one of <1> to <13>, in which the host cell is a CHO-DG44 cell, a CHO-K1 cell, a CHO-DXB11 cell, a CHOpro3⁻ cell, or an established cell line derived from these cells.
<17-1> The method for producing cells according to <1>, in which
   the heteromultimeric protein is a bispecific antibody consisting of a first H-chain, a second H-chain, and an L-chain that is common to the first H-chain and the second H-chain,
   the subunit X is the L-chain, and the subunit Y is the first H-chain and the second H-chain, and
   in which the vector set is a vector set below,
   in which
   the vector set is the set of the first expression vector and the second expression vector,
   both the first expression vector and the second expression vector contain an expression cassette of the L-chain,
   one of the first expression vector and the second expression vector contains an expression cassette of the first H-chain, and
   one of the first expression vector and the second expression vector contains an expression cassette of the second H-chain.
<17-2> A method for producing cells that produce a bispecific antibody consisting of a first H-chain, a second H-chain, and an L-chain common to the first H-chain and the second H-chain, including:
   introducing a vector set into a host cell; and
   selecting cells that produce a bispecific antibody from the host cell into which the vector set has been introduced,
   in which
   the vector set is a set of a first expression vector and a second expression vector,
   both the first expression vector and the second expression vector contain an expression cassette of the L-chain,
   one of the first expression vector and the second expression vector contains an expression cassette of the first H-chain, and
   one of the first expression vector and the second expression vector contains an expression cassette of the second H-chain.
<17-3> The method for producing cells according to <17-1> or <17-2>, in which
   one of the first expression vector and the second expression vector contains the expression cassette of the first H-chain and the expression cassette of the second H-chain.
<17-4> The method for producing cells according to any one of <17-1> to <17-3>, in which
   the first expression vector contains the expression cassette of the L-chain, the expression cassette of the first H-chain, and the expression cassette of the second H-chain, and
   the second expression vector contains the expression cassette of the L-chain.
<17-5> The method for producing cells according to any one of <17-1> to <17-4>, in which
   the bispecific antibody is a tetramer consisting of one first H-chain, one second H-chain, and two L-chains.
<18-1> The method for producing cells according to any one of <17-1> to <17-5>, in which
   the first expression vector and the second expression vector each contain one expression cassette of the L-chain,
   one of the first expression vector and the second expression vector contains one expression cassette of the first H-chain, and
   one of the first expression vector and the second expression vector contains one expression cassette of the second H-chain.
<18-2> The method for producing cells according to <1>, in which
   the heteromultimeric protein is a bispecific antibody consisting of the H-chain, the L-chain, and a scFv-Fc,
   the subunit X is the L-chain and scFv-Fc, and the subunit Y is the H-chain, and
   the vector set is a vector set A below,
   in which
   the vector set A is a set of the first expression vector and the second expression vector,
   both the first expression vector and the second expression vector contain an expression cassette of the L-chain and an expression cassette of the scFv-Fc, and
   one of the first expression vector and the second expression vector contains an expression cassette of the H-chain.
<18-3> The method for producing cells according to <1>, in which
   the heteromultimeric protein is a bispecific antibody consisting of the H-chain, the L-chain, and the scFv-Fc,
   the subunit X is the scFv-Fc, and the subunit Y is the H-chain and the L-chain, and
   the vector set is a vector set B below,
   in which
   the vector set B is a set of the first expression vector and the second expression vector,
   both the first expression vector and the second expression vector contain the expression cassette of the scFv-Fc,
   one of the first expression vector and the second expression vector contains the expression cassette of the H-chain, and
   one of the first expression vector and the second expression vector contains the expression cassette of the L-chain.
<18-4> A method for producing cells that produce a bispecific antibody consisting of an H-chain, an L-chain and scFv-Fc, including:
   introducing a vector set A or B into a host cell; and
   selecting cells that produce a bispecific antibody from the host cell into which the vector set A or B has been introduced,
   in which
   the vector set A is a set of a first expression vector and a second expression vector,
   both the first expression vector and the second expression vector contain an expression cassette of the L-chain and an expression cassette of the scFv-Fc, and
   one of the first expression vector and the second expression vector contains an expression cassette of the H-chain,
   in which
   the vector set B is a set of the first expression vector and the second expression vector,
   both the first expression vector and the second expression vector contain the expression cassette of the scFv-Fc,
   one of the first expression vector and the second expression vector contains the expression cassette of the H-chain, and
   one of the first expression vector and the second expression vector contains the expression cassette of the L-chain.
<18-5> The method for producing cells according to any one of <18-2> to <18-4>, in which the bispecific antibody is a trimer consisting of one H-chain, one L-chain, and one scFv-Fc.
<19> The method for producing cells according to any one of <17-1> to <17-5> and <18-1> to <18-5>, in which
   all of the expression cassettes of the subunits constituting the bispecific antibody contain promoters of the same type.
<20> The method for producing cells according to <19>, in which
   the promoter is an hEF-1α promoter.
<21> The method for producing cells according to any one of <17-1> to <17-5>, <18-1> to <18-5>, <19>, and <20>, in which
   the at least one of the expression cassettes of the subunits constituting the bispecific antibody contains a coding sequence of a fibronectin secretion leader, and
   in the expression cassettes containing the coding sequence of the fibronectin secretion leader, a coding sequence of the subunits constituting the bispecific antibody is arranged downstream of the coding sequence of the fibronectin secretion leader in the same reading frame.
<22> The method for producing cells according to any one of <17-1> to <17-5>, <18-1> to <18-5>, <19>, and <20>, in which
   all of the expression cassettes of the subunits constituting the bispecific antibody contain a coding sequence of a fibronectin secretion leader, and
   a coding sequence of the subunits constituting the bispecific antibody is arranged downstream of the coding sequence of the fibronectin secretion leader in the same reading frame.
<23> The method for producing cells according to any one of <17-1> to <17-5>, <18-1> to <18-5>, and <19> to <22>, in which
   the first expression vector further contains an expression cassette of a first selection marker, and
   the second expression vector further contains an expression cassette of a second selection marker.
<24> The method for producing cells according to <23>, in which
   the first expression vector contains the expression cassette of the L-chain, the expression cassette of the first H-chain, the expression cassette of the second H-chain, and the expression cassette of the first selection marker, and
   the second expression vector contains the expression cassette of the L-chain and the expression cassette of the second selection marker.
<25> The method for producing cells according to <24>, in which
   the first selection marker and the second selection marker are selection markers different from each other, and
   the first selection marker is a dihydrofolate reductase gene or a glutamine synthetase gene.
<26> The method for producing cells according to <24>, in which
   the first selection marker is a dihydrofolate reductase gene or a glutamine synthetase gene, and
   the second selection marker is an antibiotic resistance gene.
<27> The method for producing cells according to any one of <17-1> to <17-5>, <18-1> to <18-5>, and <19> to <26>, in which the host cell is a mammalian cell.
<28> The method for producing cells according to any one of <17-1> to <17-5>, <18-1> to <18-5>, and <19> to <26>, in which the host cell is a Chinese hamster ovary cell (CHO cell).
<29> The method for producing cells according to any one of <17-1> to <17-5>, <18-1> to <18-5>, and <19> to <26>, in which the host cell is a CHO-DG44 cell, a CHO-K1 cell, a CHO-DXB11 cell, a CHOpro3⁻ cell, or an established cell line derived from these cells.
<30> A method for producing a heteromultimeric protein, including culturing cells that were produced by the method for producing cells according to any one of <1> to <16>.
<31> A method for producing a bispecific antibody, including culturing cells that were produced by the method for producing cells according to any one of <17-1> to <17-5>, <18-1> to <18-5>, and <19> to <29>.
<32> A vector set that expresses a heteromultimeric protein consisting of n types of subunits (wherein, n is an integer of 2 or higher), in which:
   the vector set is a set of a first expression vector and a second expression vector,
   an expression cassette of at least one subunit X that is a portion of the n types of subunits is contained in both the first expression vector and the second expression vector, and
   expression cassettes of subunits Y remaining after the subunit X is removed from the n types of subunits are contained in one of the first expression vector and the second expression vector for each type.
<33> The vector set according to <32>, in which
   the vector set is the set of the first expression vector and the second expression vector,
   the expression cassette of the at least one subunit X that is a portion of the n types of subunits is contained in both the first expression vector and the second expression vector, and
   all of the expression cassettes of the subunits Y remaining after the subunit X is removed from the n types of subunits are contained in one of the first expression vector and the second expression vector.
<34> The vector set according to <32> or <33>, in which
   the heteromultimeric protein consisting of the n types of subunits is a heteromultimeric protein consisting of two or more types and six or fewer types of subunits, and
   the at least one subunit X is one type, two types, or three types of subunits X.
<35> The vector set according to <32> or <33>, in which
   the heteromultimeric protein consisting of the n types of subunits is a heteromultimeric protein consisting of three or four types of subunits, and
   the at least one subunit X is one type, two types, or three types of subunits X.
<36> The vector set according to <32> or <33>, in which
   the heteromultimeric protein consisting of the n types of subunits is a heteromultimeric protein consisting of three types of subunits, and
   the at least one subunit X is one type or two types of subunits X.
<37> The vector set according to any one of <32> to <36>, in which
   the heteromultimeric protein is an antibody.
<38> The vector set according to any one of <32> to <36>, in which
   the heteromultimeric protein is a bispecific antibody.
<39> The vector set according to any one of <32> to <38>, in which
   all of the expression cassettes of the subunits constituting the heteromultimeric protein contain promoters of the same type.
<40> The vector set according to <39>, in which
   the promoter is an hEF-1α promoter.
<41> The vector set according to any one of <32> to <40>, in which
   at least one of the expression cassettes of the subunits constituting the heteromultimeric protein contains a coding sequence of a fibronectin secretion leader, and
   in the expression cassettes containing the coding sequence of the fibronectin secretion leader, a coding sequence of the subunits constituting the heteromultimeric protein is arranged downstream of the coding sequence of the fibronectin secretion leader in the same reading frame.
<42> The vector set according to any one of <32> to <40>, in which
   all of the expression cassettes of the subunits constituting the heteromultimeric protein contain a coding sequence of a fibronectin secretion leader, and
   a coding sequence of the subunits constituting the heteromultimeric protein is arranged downstream of the coding sequence of the fibronectin secretion leader in the same reading frame.
<43> The vector set according to any one of <32> to <42>, in which
   the first expression vector further contains an expression cassette of a first selection marker, and
   the second expression vector further contains an expression cassette of a second selection marker.
<44> The vector set according to <43>, in which
   the first selection marker and the second selection marker are selection markers different from each other,
   the first selection marker is at least one selected from the group consisting of a dihydrofolate reductase gene, a glutamine synthetase gene, and an antibiotic resistance gene, and
   the second selection marker is at least one selected from the group consisting of a dihydrofolate reductase gene, a glutamine synthetase gene, and an antibiotic resistance gene.
<45-1> The vector set according to <32>, in which
   the heteromultimeric protein is a bispecific antibody consisting of a first H-chain, a second H-chain, and an L-chain that is common to the first H-chain and the second H-chain,
   the subunit X is the L-chain, and the subunit Y is the first H-chain and the second H-chain, and
   both the first expression vector and the second expression vector contain an expression cassette of the L-chain,
   one of the first expression vector and the second expression vector contains an expression cassette of the first H-chain, and
   one of the first expression vector and the second expression vector contains an expression cassette of the second H-chain.
<45-2> A vector set that expresses a bispecific antibody consisting of a first H-chain, a second H-chain, and an L-chain common to the first H-chain and the second H-chain, in which
   the vector set is a set of a first expression vector and a second expression vector,
   both the first expression vector and the second expression vector contain an expression cassette of the L-chain,
   one of the first expression vector and the second expression vector contains an expression cassette of the first H-chain, and
   one of the first expression vector and the second expression vector contains an expression cassette of the second H-chain.
<45-3> The vector set according to <45-1> or <45-2>, in which
   one of the first expression vector and the second expression vector contains the expression cassette of the first H-chain and the expression cassette of the second H-chain.
<45-4> The vector set according to any one of <45-1> to <45-3>, in which
   the first expression vector contains the expression cassette of the L-chain, the expression cassette of the first H-chain, and the expression cassette of the second H-chain, and
   the second expression vector contains the expression cassette of the L-chain.
<45-5> The vector set according to any one of <45-1> to <45-4>, in which
   the bispecific antibody is a tetramer consisting of one first H-chain, one second H-chain, and two L-chains.
<46-1> The vector set according to any one of <45-1> to <45-5>, in which
   the first expression vector and the second expression vector each contain one expression cassette of the L-chain,
   one of the first expression vector and the second expression vector contains one expression cassette of the first H-chain, and
   one of the first expression vector and the second expression vector contains one expression cassette of the second H-chain.
<46-2> The vector set according to <32>, in which
   the heteromultimeric protein is a bispecific antibody consisting of an H-chain, an L-chain, and scFv-Fc,
   the subunit X is the L-chain and the scFv-Fc, and the subunit Y is the H-chain,
   both the first expression vector and the second expression vector contain an expression cassette of the L-chain and an expression cassette of the scFv-Fc, and
   one of the first expression vector and the second expression vector contains an expression cassette of the H-chain.
<46-3> The vector set according to <32>, in which
   the heteromultimeric protein is a bispecific antibody consisting of the H-chain, the L-chain, and the scFv-Fc,
   the subunit X is the scFv-Fc, and the subunit Y is the H-chain and the L-chain,
   both the first expression vector and the second expression vector contain the expression cassette of the scFv-Fc,
   one of the first expression vector and the second expression vector contains the expression cassette of the H-chain, and
   one of the first expression vector and the second expression vector contains the expression cassette of the L-chain.
<46-4> A vector set that expresses a bispecific antibody consisting of an H-chain, an L-chain, and scFv-Fc, in which
   the vector set is a set of a first expression vector and a second expression vector,
   both the first expression vector and the second expression vector contain an expression cassette of the L-chain and an expression cassette of the scFv-Fc, and
   one of the first expression vector and the second expression vector contains an expression cassette of the H-chain.
<46-5> A vector set that expresses a bispecific antibody consisting of an H-chain, an L-chain, and scFv-Fc, in which
   the vector set is a set of the first expression vector and the second expression vector,
   both the first expression vector and the second expression vector contain the expression cassette of the scFv-Fc,
   one of the first expression vector and the second expression vector contains the expression cassette of the H-chain, and
   one of the first expression vector and the second expression vector contains the expression cassette of the L-chain.
<46-6> The vector set according to any one of <46-2> to <46-5>, in which
   the bispecific antibody is a trimer consisting of the H-chain, the L-chain, and the scFv-Fc.
<47> The vector set according to any one of <45-1> to <45-5> and <46-1> to <46-6>, in which
   all of the expression cassettes of the subunits constituting the bispecific antibody contain promoters of the same type.
<48> The vector set according to <47>, in which
   the promoter is an hEF-1α promoter.
<49> The vector set according to any one of <45-1> to <45-5>, <46-1> to <46-6>, <47>, and <48>, in which
   at least one of the expression cassettes of the subunits constituting the bispecific antibody contains a coding sequence of a fibronectin secretion leader, and
   in the expression cassettes containing the coding sequence of the fibronectin secretion leader, a coding sequence of the subunits constituting the bispecific antibody is arranged downstream of the coding sequence of the fibronectin secretion leader in the same reading frame.
<50> The vector set according to any one of <45-1> to <45-5>, <46-1> to <46-6>, <47>, and <48>, in which
   all of the expression cassettes of the subunits constituting the bispecific antibody contains a coding sequence of a fibronectin secretion leader, and
   a coding sequence of the subunits constituting the bispecific antibody is arranged downstream of the coding sequence of the fibronectin secretion leader in the same reading frame.
<51> The vector set according to any one of <45-1> to <45-5>, <46-1> to <46-6>, and <47> to <50>, in which
   the first expression vector further contains an expression cassette of a first selection marker, and
   the second expression vector further contains an expression cassette of a second selection marker.
<52> The vector set according to <51>, in which
   the first expression vector contains the expression cassette of the L-chain, the expression cassette of the first H-chain, the expression cassette of the second H-chain, and the expression cassette of the first selection marker, and
   the second expression vector contains the expression cassette of the L-chain and the expression cassette of the second selection marker.
<53> The vector set according to <52>, in which
   the first selection marker and the second selection marker are selection markers different from each other, and
   the first selection marker is a dihydrofolate reductase gene or a glutamine synthetase gene.
<54> The vector set according to <52>, in which
   the first selection marker is a dihydrofolate reductase gene or a glutamine synthetase gene, and
   the second selection marker is an antibiotic resistance gene.
<55> A mammalian cell that is transfected with the vector set according to any one of <32> to <44>, <45-1> to <45-5>, <46-1> to <46-6>, and <47> to <54>.
<56> A Chinese hamster ovary cell (CHO cell) that is transfected with the vector set according to any one of <32> to <44>, <45-1> to <45-5>, <46-1> to <46-6>, and <47> to <54>.
<57> A mammalian cell that is transfected with the vector set according to any one of <45-1> to <45-5>, <46-1> to <46-6>, and <47> to <54>, in which
   when the amount of mRNA of the first H-chain is 1, the amount of mRNA of the second H-chain is 0.5 to 2, and the amount of mRNA of the L-chain is 2 to 10.
<58> A mammalian cell that is transfected with the vector set according to any one of <45-1> to <45-5>, <46-1> to <46-6>, and <47> to <54>, in which
   when the amount of mRNA of the first H-chain is 1, the amount of mRNA of the second H-chain is 0.625 to 1.6, and the amount of mRNA of the L-chain is 3 to 8.
<59> A CHO cell that is transfected with the vector set according to any one of <45-1> to <45-5>, <46-1> to <46-6>, and <47> to <54>, in which
   when the amount of mRNA of the first H-chain is 1, the amount of mRNA of the second H-chain is 0.5 to 2, and the amount of mRNA of the L-chain is 2 to 10.
<60> A CHO cell that is transfected with the vector set according to any one of <45-1> to <45-5>, <46-1> to <46-6>, and <47> to <54>, in which
   when the amount of mRNA of the first H-chain is 1, the amount of mRNA of the second H-chain is 0.625 to 1.6, and the amount of mRNA of the L-chain is 3 to 8.
<61> A method for producing a cell pool, including introducing the vector set according to any one of <32> to <44>, <45-1> to <45-5>, <46-1> to <46-6>, and <47> to <54> into a host cell.
<62> The method for producing a cell pool according to <61>, in which the host cell is a mammalian cell.
<63> The method for producing a cell pool according to <61>, in which the host cell is a Chinese hamster ovary cell (CHO cell).
<64> The method for producing a cell pool according to <61>, in which the host cell is a CHO-DG44 cell, a CHO-K1 cell, a CHO-DXB11 cell, a CHOpro3⁻ cell, or an established cell line derived from these cells.

### Advantageous Effects of Invention

According to an embodiment of the present disclosure, provided is a method for producing cells excellent in productivity of a heteromultimeric protein or a bispecific antibody.

According to an embodiment of the present disclosure, provided is a method for producing a heteromultimeric protein or a bispecific antibody excellent in productivity.

According to an embodiment of the present disclosure, provided is a vector set used for producing cells that produce a heteromultimeric protein or a bispecific antibody.

According to an embodiment of the present disclosure, provided are mammalian cells and CHO cells that produce a heteromultimeric protein or a bispecific antibody.

According to an embodiment of the present disclosure, provided is a method for producing a cell pool used for selecting cells that produce a heteromultimeric protein or a bispecific antibody.

### Brief Description of Drawings

[Figure 1] Figure 1 shows a vector map of an expression vector 1 constructed in Examples.
[Figure 2] Figure 2 shows a vector map of an expression vector 2 constructed in Examples.
[Figure 3] Figure 3 shows a vector map of an expression vector 3 constructed in Examples.
[Figure 4] Figure 4 shows a vector map of an expression vector 4 constructed in Examples.
[Figure 5] Figure 5 shows a conceptual diagram illustrating a configuration of bispecific antibodies (BiAb1 and BiAb2) produced in Examples.

### Description of Embodiments

Hereinafter, embodiments of the present disclosure will be described. The description and Examples are illustrative of the embodiments and not intended to limit the scope of the embodiments. The mechanism of action to be described in the present disclosure includes presumptions, and whether they are right or wrong does not limit the scope of the embodiments.

A numerical value range expressed using "to" in the present disclosure indicates a range including the numerical values written before and after "to" as the minimum value and the maximum value, respectively.

In stepwise numerical value ranges shown in the present disclosure, the upper limit value or the lower limit value shown in one numerical value range can be replaced with the upper limit value or the lower limit value shown in another stepwise numerical value range. In addition, in a numerical value range shown in the present disclosure, the upper limit value or the lower limit value in the numerical value range can be replaced with the values shown in Examples.

In the present disclosure, each component may contain multiple types of substances corresponding thereto. In the present disclosure, in a case where multiple types of substances corresponding to each component are present in a composition, the amount of each component in the composition means the total amount of the multiple types of substances present in the composition, unless otherwise stated.

In the present disclosure, the term nucleic acid includes any nucleic acids (e.g**.,** DNA, RNA, analogs thereof, natural products, and artificial products), and nucleic acids in which low molecular compounds, groups (e.g.**,** methyl groups), molecules other than nucleic acids, structures, and the like are attached to any nucleic acids. Nucleic acids may be single-stranded or double-stranded.

In the present disclosure, a vector is a substance that has the effect of transporting a foreign nucleic acid into a cell, and is a nucleic acid itself. An expression vector refers to a vector that expresses a polypeptide based on the sequence of its nucleic acid. The sizes and base sequences of vectors and expression vectors are not limited. Vectors and expression vectors are preferably double-stranded DNA.

In the present disclosure, a polypeptide refers to a molecule in which amino acids are linked by peptide bonds. The number of amino acid residues in a polypeptide is not limited, and the term polypeptide includes proteins. Polypeptides in the present disclosure desirably have six or more amino acid residues. Polypeptides include polypeptides in which amino acids have undergone post-translational modification. Examples of post-translational modifications of amino acids include phosphorylation, methylation, and acetylation.

In the present disclosure, three-letter codes and one-letter codes determined by IUPAC-IUBMB Joint Commission on Biochemical Nomenclature (IUPAC-IUBMB JCBN) are used to express amino acids. Amino acids referred to in the present disclosure are L-amino acids, unless otherwise stated.

In the present disclosure, the heteromultimeric protein refers to a protein in which at least two types of subunits are aggregated. One subunit refers to a single polypeptide constituting a multimeric protein. One polypeptide constituting a multimeric protein and in which a plurality of polypeptides are linked by one or more peptide linkers corresponds to one subunit. In other words, the heteromultimeric protein refers to a protein made up of at least two types of coding sequences that are expressed independently of each other. The heteromultimeric protein consisting of n types of subunits is expressed by n types of coding sequences that are expressed independently of each other (wherein, n is an integer of 2 or higher).

Examples of the heteromultimeric protein include an antibody, an Fc fusion protein, an enzyme, an interleukin, a cytokine, a chemokine, a peptide hormone, a growth factor, a transcription factor, a receptor, a receptor fragment, a therapeutic protein, a virus, a virus-like particle, and a vaccine.

In the present disclosure, the antibody is not limited to immunoglobulin, and may be any molecule as long as it binds to an antigen. In the present disclosure, the term antibody encompasses an antibody fragment and an antigen-binding molecule. The antibody includes both monovalent and multivalent antibodies. The antibody includes an immunoglobulin molecule per se (intact immunoglobulin), a Fab antibody, a Fab' antibody, F(ab')₂ antibody, a single-stranded antibody (scFv), a diabody, and variants thereof. The antibody has an antigen-recognition site called paratope, and the paratope binds to an antigen.

General IgG contains two heavy chains (HC) whose amino acid sequences are identical and two light chains (LC) whose amino acid sequences are identical, and has a structure in which both the HCs are connected by a disulfide bond, and the HC and LC are connected by a disulfide bond. The HC contains a variable region (VH) and a constant region (CH1, CH2, and CH3), and the LC contains a variable region (VL) and a constant region (CL).

Examples of the antibody include a human antibody that has a human variable region and a human constant region; a mouse antibody that has a mouse variable region and a mouse constant region; a chimeric antibody in which regions derived from a plurality of animals are combined; a humanized antibody that has a mouse variable region and a human constant region; and a humanized antibody that has a camelid heavy chain variable region and a human Fc region.

Examples of the antibody include a bispecific antibody and a multispecific antibody. The bispecific antibody refers to an antibody capable of simultaneously binding to two epitopes. The multispecific antibody refers to an antibody capable of simultaneously binding to three or more epitopes. The structure of the bispecific antibody and the number of epitope-binding sites are not limited. The structure of the multispecific antibody and the number of epitope-binding sites are not limited.

Examples of the bispecific antibody include a bispecific antibody consisting of a first H-chain, a second H-chain, and an L-chain common to the first **H-**chain and the second H-chain. Note that in the present disclosure, the L-chain common to the two H-chains refers to one L-chain that pairs with the two H-chains. One example of this bispecific antibody is a tetramer in which one first H-chain, one second H-chain, and two L-chains are connected to each other by disulfide bonds. The first H-chain, the second H-chain, and the L-chain each need only to contain at least a region for recognizing an antigen and a region for forming an antibody.

Further examples of the bispecific antibody include a bispecific antibody consisting of an H-chain, an L-chain, and scFv-Fc. The scFv is a fusion protein of an immunoglobulin heavy chain variable region and a light chain variable region, and the scFv-Fc is a fusion protein of scFv and Fc region. One example of this bispecific antibody is a trimer in which one H-chain, one L-chain, and one scFv-Fc are connected to each other by disulfide bonds.

The antigen and structure of the bispecific antibody are not limited as long as it has an arm that recognizes a first antigen and an arm that recognizes a second antigen. Examples of the bispecific antibody include emicizumab, blinatumomab, vanucizumab, istiratumab, pasotuxizumab, duligotuzumab, duvortuxizumab, and faricimab.

An embodiment of the present disclosure is a method for producing cells that produce a heteromultimeric protein. The method for producing cells includes introducing a vector set of a first expression vector and a second expression vector into a host cell, and selecting cells that produce a heteromultimeric protein from the host cell into which the vector set has been introduced.

In the present disclosure, the phrase "vector set" means a set of the first expression vector and the second expression vector.

In the present disclosure, when describing matters common to the first expression vector and the second expression vector, the first expression vector and the second expression vector are collectively referred to as "expression vector".

The host cell may be a prokaryotic cell or a eukaryotic cell. Examples of the prokaryotic cell include a bacterial cell. Examples of the eukaryotic cell include a yeast, an insect cell, and a mammalian cell. The host cell is preferably a eukaryotic cell, and more preferably a mammalian cell.

Examples of the bacterial cells include Gram-negative bacterial cells such as E. coli, Salmonella typhimurium, Serratia marcescens, Pseudomonas putida, Pseudomonas aeruginosa; and Gram-positive bacterial cells such as Bacillus subtilis. Preferred bacterial cells are Enterobacteriaceae, and more preferably E. coli, especially B strain or K12 strain.

Examples of the yeast include Saccharomyces cerevisiae, Pichia pastoris, and Hansenula polymorpha.

Examples of the insect cell include BmN cells derived from Bombyx mori, Sf9 cells and Sf21 cells derived from Spodoptera frugiperda, S2 cells derived from Drosophila melanogaster, and Pv11 cells derived from Polypedilum vanderplanki.

Examples of the mammalian cell include Chinese hamster ovary cells (CHO cells), baby hamster kidney cells (BHK cells), human embryonic kidney cell lines (e.g., HEK293 cells), human retinoblastoma-derived cell lines (e.g., PER.C6 cells), and mouse myeloma cell lines (e.g., NS0 cells and SP2/0 cells).

Preferred host cells are Chinese hamster ovary cells (CHO cells). Examples of the CHO cell include CHO-DG44 cells, CHO-K1 cells, CHO-DXB11 cells, CHOpro3⁻ cells, and established cell lines derived from these cells.

Examples of the means of introducing the expression vector into the host cell include electroporation, lipofection, microinjection, and cell infection with a viral vector. Electroporation is preferable from the viewpoints of high safety, high transfection efficiency, and low cytotoxicity. Further, from the viewpoint of homogeneous introduction into cells and chromosomes without bias depending on the type of expression vector when introducing multiple types of expression vectors into cells, electroporation is also preferable.

The order of introduction of the first expression vector and the second expression vector into the host cell is not limited. The first expression vector and the second expression vector may be introduced together or separately into the host cell. It is preferable to introduce the first expression vector and the second expression vector together into the host cell, from the viewpoint of shortening the steps and period of time required to produce cells of interest.

When introducing the first expression vector and the second expression vector together into the host cell, a vector solution containing both the expression vectors is prepared. The molar concentration ratio of the first expression vector and the second expression vector in this vector solution is, for example, 1:5 to 5:1, 1:2 to 2:1, or 1:1.

When introducing the first expression vector and the second expression vector separately into the host cell, a vector solution containing the first expression vector and a vector solution containing the second expression vector are prepared. The molar concentration ratio of both the expression vectors in the vector solutions is, for example, 1:5 to 5:1, 1:2 to 2:1, or 1:1. When both the vectors are introduced into the host cell by separately bringing the vector solutions into contact with the host cell, the molar ratio of the first expression vector and the second expression vector to be brought into contact with the host cell is preferably 1:5 to 5:1, 1:2 to 2:1, or 1:1, for example.

The sizes of the first expression vector and the second expression vector are not limited.

The size of the first expression vector is, for example, 400 bp (base pair) to 50,000 bp, 600 bp to 20,000 bp, or 800 bp to 10,000 bp.

The size of the second expression vector is, for example, 400 bp (base pair) to 50,000 bp, 600 bp to 20,000 bp, or 800 bp to 10,000 bp.

The expression vector that has been introduced into the host cell may be integrated into the genome of the host cell, may be integrated into an extrachromosomal element such as plasmid, or may be contained in the cell as an independent extrachromosomal element. The expression vector is preferably integrated into the genome of the host cell, from the viewpoint of allowing the heteromultimeric protein of interest to be expressed for a long period of time.

Selecting cells that produce a heteromultimeric protein from the host cell into which the vector set has been introduced is performed by, for example, setting criteria for the expression level and/or purity of the heteromultimeric protein and selecting the cells that reach the criteria; and selecting the cells in which the expression level and/or purity of the heteromultimeric protein is relatively high. Specifically, for example, the following (a) to (d) are conducted.

(a) Adding a selective agent to a medium of host cells.
(b) Making the host cells into a single cell.
(c) Collecting a portion of a culture solution of the single cell and measuring the expression level and/or purity of the heteromultimeric protein.
(d) Selecting the cells in which the expression level and/or purity of the heteromultimeric protein is equal to or greater than the reference value, and/or selecting the cells in which the expression level and/or purity of the heteromultimeric protein is relatively high.

The expression level of the heteromultimeric protein is the amount of the heteromultimeric protein itself.

The purity of the heteromultimeric protein refers to the proportion of the heteromultimeric protein of interest to the total amount of multiple types of proteins. The expression level and/or purity of proteins can be measured by a known method. For example, the amount of protein separated by electrophoresis or the like is detected by a known method such as a fluorescence method, and absorbance measurement. Measurement equipment such as icIEF analysis instrument Maurice (Protein Simple) can be used. In the expression of heteromultimeric proteins, multimeric proteins not in their original shapes (e.g., multimeric proteins lacking some subunits, or multimeric proteins in which a subunit is replaced by another subunit) may occur, but it is desirable that the proportion of multimeric proteins not in their original shapes be low.

The cells that produce a heteromultimeric protein may be transiently expressing cells or stably expressing cells, and are preferably stably expressing cells.

An embodiment of the present disclosure is a vector set used for producing cells that produce a heteromultimeric protein. The vector set is a set of the first expression vector and the second expression vector, and the two expression vectors contain all of the expression cassettes of the subunits constituting a heteromultimeric protein. The heteromultimeric protein consisting of n types of subunits is expressed from n types of expression cassettes in which the coding sequences of the subunits are different from each other. The vector set contains n types of expression cassettes in which the coding sequences of the subunits are different from each other.

The expression cassette of the subunit contains all nucleic acids required for the expression of the subunit. The size and the base sequence of the expression cassette of the subunit are not limited. The size of the expression cassette of the subunit is, for example, 400 bp (base pair) to 4000 bp, 600 bp to 3000 bp, or 800 bp to 2000 bp.

In the construction of the vector set, subunits constituting heteromultimeric proteins are divided into two groups of subunit X and subunit Y.

The subunit X is a part of subunits constituting heteromultimeric proteins, and is a subunit whose expression is to be enhanced, for example. The subunit X is a part, but not all, of subunits constituting heteromultimeric proteins. The subunit X may be in one type, or two or more types. In the heteromultimeric protein consisting of n types of subunits, the number of types of the subunit X is an integer of 1 or higher and (n-1) or smaller.

The subunit Y is subunits remaining after the subunit X is removed from the subunits constituting the heteromultimeric protein. The subunit Y may be in one type, or two or more types.

The expression cassette of the subunit X is contained in both the first expression vector and the second expression vector. In other words, both the first expression vector and the second expression vector contain the expression cassette of the subunit X. The subunit X is expressed from both the first expression vector and the second expression vector.

For example, when the subunit X is composed of three types of X1, X2, and X3, the first expression vector contains the expression cassette of X1, the expression cassette of X2, and the expression cassette of X3, and the second expression vector contains the expression cassette of X1, the expression cassette of X2, and the expression cassette of X3.

The number of the expression cassettes of at least one subunit X that the first expression vector contains may be one, or two or more for each subunit type. When the subunit X is composed of two or more types, the number of the expression cassettes of the subunit X that the first expression vector contains may be the same or different between subunit types.

For example, when the subunit X is composed of three types of X1, X2, and X3, the number of the expression cassettes of X1, the number of the expression cassettes of X2, and the number of the expression cassettes of X3 that the first expression vector contains are independent from each other; and the numbers may all be the same, or may be different from each other.

The number of the expression cassettes of at least one subunit X that the second expression vector contains may be one, or two or more for each subunit type. When the subunit X is composed of two or more types, the number of the expression cassettes of the subunits X that the second expression vector contains may be the same or different between subunit types.

For example, when the subunit X is composed of three types of X1, X2, and X3, the number of the expression cassettes of X1, the number of the expression cassettes of X2, and the number of the expression cassettes of X3 that the second expression vector contains are independent from each other; and the numbers may all be the same, or may be different from each other.

One example of embodiments includes a form in which the first expression vector contains one expression cassette of at least one subunit X for each type, and the second expression vector contains one expression cassette of at least one subunit X for each type.

The expression cassette of at least one subunit Y is contained in one of the first expression vector and the second expression vector for each type. At least one subunit Y is expressed from one of the first expression vector and the second expression vector for each type. When the subunit Y is composed of two or more types, one of the first expression vector and the second expression vector may contain all of the expression cassettes of the subunit Y, or the first expression vector and the second expression vector may contain the expression cassettes of the subunit Y divided for each subunit type. For example, the subunit Y is composed of three types of Y1, Y2, and Y3, the form may be any of the following:
a form in which only the first expression vector contains the expression cassette of Y1, the expression cassette of Y2, and the expression cassette of Y3;
a form in which the first expression vector contains the expression cassette of Y1, and the second expression vector contains the expression cassette of Y2, and the expression cassette of Y3;
a form in which the first expression vector contains the expression cassette of Y2, and the second expression vector contains the expression cassette of Y1, and the expression cassette of Y3; and
a form in which the first expression vector contains the expression cassette of Y3, and the second expression vector contains the expression cassette of Y1, and the expression cassette of Y2.

When the first expression vector contains the expression cassettes of the subunit Y, the number of the expression cassettes of at least one subunit Y that the first expression vector contains may be one, or two or more for each subunit type. When the first expression vector contains two or more types of expression cassettes of the subunit Y, the number of the expression cassettes of the subunits Y that the first expression vector contains may be the same or different between subunit types.

For example, when the subunit Y that the first expression vector contains is composed of three types of Y1, Y2, and Y3, the number of the expression cassettes of Y1, the number of the expression cassettes of Y2, and the number of the expression cassettes of Y3 that the first expression vector contains are independent from each other; and the numbers may all be the same, or may be different from each other.

When the second expression vector contains the expression cassettes of the subunit Y, the number of the expression cassettes of at least one subunit Y that the second expression vector contains may be one, or two or more for each subunit type. When the second expression vector contains two or more types of expression cassettes of the subunit Y, the number of the expression cassettes of the subunits Y that the second expression vector contains may be the same or different between subunit types.

For example, when the subunit Y that the second expression vector contains is composed of three types of Y1, Y2, and Y3, the number of the expression cassettes of Y1, the number of the expression cassettes of Y2, and the number of the expression cassettes of Y3 that the second expression vector contains are independent from each other; and the numbers may all be the same, or may be different from each other.

One example of embodiments includes a form in which one of the first expression vector and the second expression vector contains one each of all types of the expression cassettes of the subunit Y.

Another example of embodiments includes a form in which the first expression vector and the second expression vector contain expression cassettes of the subunit Y, one for each type.

One example of embodiments of the vector set includes a vector set in which the expression cassette of at least one subunit X is contained in both the first expression vector and the second expression vector, and all of the expression cassettes of at least one subunit Y are contained in one of the first expression vector and the second expression vector.

In other words, in the vector set above, both the first expression vector and the second expression vector contain the expression cassettes of the subunit X, and one of the first expression vector and the second expression vector contains the expression cassettes of the subunit Y.

One example of embodiments of the vector set includes a vector set in which the first expression vector contains one each of all types of the expression cassettes of the subunit X, the second expression vector contains one each of all types of the expression cassettes of the subunit X, and one of the first expression vector and the second expression vector contains one each of all types of the expression cassettes of the subunit Y.

Examples of preferred forms using the vector set include one in which the heteromultimeric protein is an antibody. Examples of more preferred forms using the vector set include one in which the heteromultimeric protein is a bispecific antibody.

Examples of preferred forms using the vector set include one in which the heteromultimeric protein consists of two or more types and six or fewer types of subunits, and the subunit X is in one type, two types, or three types. Examples of the form include:
a form in which the heteromultimeric protein consists of two types of subunits, and the subunit X is in one type;
a form in which the heteromultimeric protein consists of three types of subunits, and the subunit X is in one type;
a form in which the heteromultimeric protein consists of three types of subunits, and the subunit X is in two types;
a form in which the heteromultimeric protein consists of four types of subunits, and the subunit X is in one type;
a form in which the heteromultimeric protein consists of four types of subunits, and the subunit X is in two types;
a form in which the heteromultimeric protein consists of five types of subunits, and the subunit X is in one type;
a form in which the heteromultimeric protein consists of six types of subunits, and the subunit X is in two types; and
a form in which the heteromultimeric protein consists of six types of subunits, and the subunit X is in three types.

Examples of more preferred forms using the vector set include one in which the heteromultimeric protein consists of three, four, or five types of subunits, and the subunit X is in one or two types. Examples of the form include:
a form in which the heteromultimeric protein consists of three types of subunits, and the subunit X is in one type;
a form in which the heteromultimeric protein consists of three types of subunits, and the subunit X is in two types;
a form in which the heteromultimeric protein consists of four types of subunits, and the subunit X is in one type;
a form in which the heteromultimeric protein consists of four types of subunits, and the subunit X is in two types; and
a form in which the heteromultimeric protein consists of five types of subunits, and the subunit X is in one type.

Examples of further preferred forms using the vector set include one in which the heteromultimeric protein consists of three or four types of subunits, and the subunit X is in one, two, or three types. Examples of the form include:
a form in which the heteromultimeric protein consists of three types of subunits, and the subunit X is in one type;
a form in which the heteromultimeric protein consists of three types of subunits, and the subunit X is in two types;
a form in which the heteromultimeric protein consists of four types of subunits, and the subunit X is in one type; and
a form in which the heteromultimeric protein consists of four types of subunits, and the subunit X is in two types.

Examples of particularly preferred forms using the vector set include one in which the heteromultimeric protein consists of three types of subunits, and the subunit X is in one or two types.

One example of forms using the vector set includes one in which the heteromultimeric protein is a bispecific antibody consisting of the first H-chain, the second H-chain, and the L-chain common to the first H-chain and the second H-chain. In this form, it is preferable that the expression level of the L-chain be equal to or greater than the total expression levels of the first H-chain and the second H-chain. In addition, in this form, it is preferable that the amount of mRNA of the L-chain be equal to or greater than the total amount of mRNA of the first H-chain and the second H-chain. The amount of mRNA can be determined by quantitative PCR, as described later. One example of embodiments of the vector set for the bispecific antibody includes a vector set below.

A vector set, in which both the first expression vector and the second expression vector contain the expression cassette of the L-chain, one of the first expression vector and the second expression vector contains the expression cassette of the first H-chain, and one of the first expression vector and the second expression vector contains the expression cassette of the second H-chain.

In one example of the vector set above, one of the first expression vector and the second expression vector contains the expression cassette of the first H-chain and the expression cassette of the second H-chain.

In another example of the vector set above, one of the first expression vector and the second expression vector contains the expression cassette of the first H-chain, and the other contains the expression cassette of the second H-chain.

To allow the expression level of the first H-chain and the expression level of the second H-chain to be equal, it is preferable that one of the first expression vector and the second expression vector contain the expression cassette of the first H-chain and the expression cassette of the second H-chain at a number ratio of 1:1.

From the viewpoint of reducing the size of the expression vector, the vector set for the bispecific antibody above is preferable such that the first expression vector and the second expression vector each contain one expression cassette of the L-chain, one of the first expression vector and the second expression vector contains one expression cassette of the first H-chain, and one of the first expression vector and the second expression vector contains one expression cassette of the second H-chain.

Examples of preferred forms of the vector set for the bispecific antibody consisting of the first H-chain, the second H-chain, and the L-chain common to the first H-chain and the second H-chain include one in which the first expression vector contains one each of the expression cassette of the L-chain, the expression cassette of the first H-chain, and the expression cassette of the second H-chain, and the second expression vector contains one expression cassette of the L-chain.

One example of the bispecific antibody consisting of the first H-chain, the second H-chain, and the L-chain common to the first H-chain and the second H-chain is a tetramer in which one first H-chain, one second H-chain, and two L-chains are aggregated.

Another example of forms using the vector set include one in which the heteromultimeric protein is a bispecific antibody consisting of the H-chain, the L-chain, and scFv-Fc. In this form, the expression level of the scFv-Fc is preferably 0.3-fold or more, and more preferably 0.5-fold or more compared to the expression level of the H-chain. Also, in this form, the amount of mRNA of the scFv-Fc is preferably 0.3-fold or more, and more preferably 0.5-fold or more compared to the amount of mRNA of the H-chain. The amount of mRNA can be determined by quantitative PCR, as described later. One example of embodiments of the vector set for the bispecific antibody includes a vector sets A and B below.

The vector set A: a vector set, in which both the first expression vector and the second expression vector contain the expression cassette of the L-chain and the expression cassette of the scFv-Fc, and one of the first expression vector and the second expression vector contains the expression cassette of the H-chain.

The vector set B: a vector set, in which both the first expression vector and the second expression vector contain the expression cassette of the scFv-Fc, one of the first expression vector and the second expression vector contains the expression cassette of the H-chain, and one of the first expression vector and the second expression vector contains the expression cassette of the L-chain.

In one example of the vector set B above, one of the first expression vector and the second expression vector contains the expression cassette of the H-chain and the expression cassette of the L-chain.

In another example of the vector set B above, one of the first expression vector and the second expression vector contains the expression cassette of the H-chain, and the other contains the expression cassette of the L-chain.

Examples of preferred forms of the vector set for the bispecific antibody consisting of the H-chain, the L-chain, and the scFv-Fc include one in which the first expression vector contains one each of the expression cassette of the H-chain, the expression cassette of the L-chain, and the expression cassette of the scFv-Fc, and the second expression vector contains one each of the expression cassette of the L-chain and the expression cassette of the scFv-Fc.

Examples of another preferred form of the vector set for the bispecific antibody consisting of the H-chain, the L-chain, and the scFv-Fc include one in which the first expression vector contains one each of the expression cassette of the H-chain, the expression cassette of the L-chain, and the expression cassette of the scFv-Fc, and the second expression vector contains one expression cassette of the scFv-Fc.

One example of the bispecific antibody consisting of the H-chain, the L-chain, and the scFv-Fc is a trimer in which one H-chain, one L-chain, and one scFv-Fc are aggregated.

The structural nucleic acids constructing expression vectors and their base sequences are not limited. Examples of the structural nucleic acids constructing expression vectors include viral vector-derived nucleic acids, non-viral vector-derived nucleic acids, and artificial nucleic acids. The structural nucleic acids may be cyclic or linear.

Examples of the viral vector-derived nucleic acids include those derived from adenoviruses, adeno-associated viruses, retroviruses, vaccinia viruses, poxviruses, lentiviruses, herpesviruses, baculoviruses, and bacteriophages.

Examples of the non-viral vector-derived nucleic acids include plasmids.

The expression vectors and expression cassettes contain elements necessary for the expression of polypeptides, depending on the type of host cell.

When the host cell is a prokaryotic cell, the expression vector may contain, for example, a replication origin, a restriction enzyme site, a transcription terminator, and a plasmid stability locus such as a cer stability sequence.

When the host cell is yeast, the expression vector may contain, for example, a promoter, a transcription terminator, a selection marker, and a replication origin.

When the host cell is an insect cell, the expression vector may contain, for example, a promoter, a transcription terminator, and a selection marker.

When the host cell is a mammalian cell, the expression vector may contain, for example, a promoter, a polyadenylation sequence (e.g., human β-globin polyA sequence, bovine growth hormone polyA sequence, SV40 early polyA sequence, and SV40 late polyA sequence), and a selection marker.

Examples of promoters that can be used for a prokaryotic cell include the promoter disclosed in J. Mol. Biol. 1986; 189(1): 113-30, a phage polymerase promoter, and an E. coli polymerase promoter. Specific examples thereof include T7A1, T7A2, T7A3, λpL, λpR, lac, lacUV5, trp, tac, trc, phoA, and rrnB.

Examples of promoters that can be used for a yeast cell include a gal promoter, an AOX1 promoter, an AOX2 promoter, a GAP promoter, a GAL1 promoter, and a GAL10 promoter.

Examples of promoters that can be used for an insect cell include a polyhedrin promoter, a P10 promoter, a viral immediate-early protein (IE-1) promoter, a MT promoter, a COPIA promoter, a CMV promoter, an RSV promoter, an SV40 promoter, a heat-shock protein promoter, an OPIE2 promoter, and an actin 5C promoter.

Examples of promoters that can be used for a mammalian cell include a virus-derived promoter, and a housekeeping gene-derived promoter. Examples of virus-derived promoters include a human CMV promoter, a rat CMV promoter, an SV40 promoter, an RSR-LTR promoter, and an HSK-TK promoter. Examples of housekeeping gene-derived promoters include an hEF-1α promoter, a Chinese hamster EF-1α promoter, a β-actin promoter, and a mouse phosphoglycerate kinase (mPGK) promoter. A preferred example of promoters that can be used for a mammalian cell is an EF-1α promoter, and more preferably an hEF-1α promoter.

Promoters contained in the expression cassette of each subunit may be the same or different in each type. In one example of embodiments, all of the expression cassettes of the subunits constituting the heteromultimeric protein contain promoters of the same type. In one preferred example of embodiments, all of the expression cassettes of the subunits constituting the heteromultimeric protein contain an hEF-1α promoter.

The expression cassettes of the subunits preferably contain a coding sequence of a secretion leader, for the purpose of facilitating transportation or secretion of the expressed polypeptides to the outside of the cell. The secretion leader is one of signal peptides, which induces the transportation or secretion of polypeptides to the outside of the cell.

When the expression cassette of the subunit contains a coding sequence of the secretion leader, the coding sequence of the secretion leader and the coding sequence of the subunit are arranged in the same reading frame. The phrase "arranged in the same reading frame" used herein refers to the fact that the coding sequences of the secretion leader and the subunit are arranged such that the secretion leader and the subunit can be expressed as one polypeptide. The expression cassette of the subunit may or may not contain a coding sequence of a linker or spacer between the coding sequence of the secretion leader and the coding sequence of the subunit.

A preferred form is one in which the coding sequence of the subunit is arranged downstream of the coding sequence of the secretion leader in the same reading frame. From the expression cassettes in this form, a recombinant polypeptide in which the secretion leader is arranged on the N-terminal side of the subunit is expressed. A more preferred form is one in which the coding sequences of the subunit are sequentially arranged downstream of the coding sequence of the secretion leader in the same reading frame. From the expression cassettes in this form, a recombinant polypeptide in which the secretion leader is bound to the N-terminal of the subunit is expressed. The term "downstream" used herein refers to the arrangement order of two coding sequences; when a coding sequence A and a coding sequence B are arranged so that the coding sequence B is transcribed after the transcription of the coding sequence **A,** it is said that the coding sequence B is arranged downstream of the coding sequence **A.**

The secretion leader of a recombinant polypeptide is generally cleaved from the recombinant polypeptide in the process of the transportation or secretion of the recombinant polypeptides.

Examples of the secretion leader include a fibronectin secretion leader, a collagen secretion leader, and an albumin secretion leader. A fibronectin secretion leader is preferable from the viewpoint of a high secretion rate of recombinant polypeptides to the outside of cells.

Examples of the fibronectin secretion leader include an amphibian fibronectin secretion leader, and a mammalian fibronectin secretion leader. Examples of the amphibian fibronectin secretion leader include a Xenopus laevis fibronectin secretion leader. Examples of the mammalian fibronectin secretion leader include fibronectin secretion leaders of human, rat, mouse, bovine, porcine, canine, feline, and Chinese hamster, and their functional equivalents. The functional equivalents of the fibronectin secretion leader have the identity of 70% or more, preferably 75% or more, more preferably 80% or more, further more preferably 90% or more, and the most preferably 95% or more to the amino acid sequence, and have the function of secreting recombinant polypeptides to the outside of cells. The sequence identity of the amino acid sequence can be calculated, for example, by using basic local alignment search tool (BLAST) (https://blast.ncbi.nlm.nih.gov/Blast.cgi).

It is preferable to select an organism from which a fibronectin secretion leader is derived, depending on the type of host cell. When the host cell is a human cell, it is preferable to use a human fibronectin secretion leader in the expression cassette. When the host cell is a rat cell, it is preferable to use a rat fibronectin secretion leader in the expression cassette. When the host cell is a CHO cell, it is preferable to use a Chinese hamster fibronectin secretion leader in the expression cassette.

Examples of embodiments of the fibronectin secretion leader include a human fibronectin secretion leader having the amino acid sequence of SEQ ID NO: 1, and a Chinese hamster fibronectin secretion leader having the amino acid sequence of SEQ ID NO: 2. One example of the coding sequence of SEQ ID NO: 1 is SEQ ID NO: 3. One example of the coding sequence of SEQ ID NO: 2 is SEQ ID NO: 4.

SEQ ID NO: 1: MLRGPGPGLLLLAVQCLGTAVPSTGA
SEQ ID NO: 2: MLRGPGPGLLLAVLCLGTAVRCTEA

SEQ ID NO: 3: 
ATGCTGAGAGGCCCTGGACCTGGACTGCTGCTGCTGGCTGTGCAGTGTCTGGGAACC

GCCGTGCCTTCTACCGGCGCC
SEQ ID NO: 4:

ATGCTCAGGGGTCCGGGACCCGGGCTGCTGCTGGCCGTCCTGTGCCTGGGGACAGCG
GTGCGCTGTACCGAAGCC

One example of embodiments includes a form in which at least one of the expression cassettes of the subunits constituting the heteromultimeric protein contains a coding sequence of a fibronectin secretion leader.

When the host cell is a human cell, it is preferable that at least one of the expression cassettes of the subunits constituting the heteromultimeric protein contain a coding sequence of a human fibronectin secretion leader.

When the host cell is a CHO cell, it is preferable that at least one of the expression cassettes of the subunits constituting the heteromultimeric protein contain a coding sequence of a Chinese hamster fibronectin secretion leader.

One example of embodiments includes a form in which all of the expression cassettes of the subunits constituting the heteromultimeric protein contain a coding sequence of a fibronectin secretion leader.

When the host cell is a human cell, it is preferable that all of the expression cassettes of the subunits constituting the heteromultimeric protein contain a coding sequence of a human fibronectin secretion leader.

When the host cell is a CHO cell, it is preferable that all of the expression cassettes of the subunits constituting the heteromultimeric protein contain a coding sequence of a Chinese hamster fibronectin secretion leader.

In one preferred example of embodiments, the expression cassettes of the subunits constituting the heteromultimeric protein contain an hEF-1α promoter, a coding sequence of a fibronectin secretion leader, a coding sequence of the subunit, and a polyA sequence, all of which are operably linked to each other.

The expression vector preferably contains expression cassettes of a selection marker for the purpose of confirming the introduction of the expression vector into host cells or producing stably expressing cells.

In the present disclosure, the selection marker is a marker that functions to select cells into which the expression vector has been introduced, and refers to a gene integrated into the expression vector and a protein encoded by the gene.

The expression cassette of the selection marker contains all nucleic acids required for the expression of the selection marker. The size and the base sequence of the expression cassette of the selection marker are not limited.

Examples of the selection marker include a protein that exhibits resistance to selective agents and a gene thereof. Examples of the selective agents include enzyme inhibitors and antibiotics.

Examples in which the selective agent is an enzyme inhibitor include the DHFR-MTX system. For the DHFR-MTX system, the selective agent is methotrexate (MTX), and the selection marker is dihydrofolate reductase (DHFR) and a gene thereof. The DHFR-MTX system is a system effective in host cells lacking the DHFR gene (e.g., CHO-DG44 cells).

Examples in which the selective agent is an enzyme inhibitor include the GS-MSX system. For the GS-MSX system, the selective agent is methionine sulfoximine (MSX), and the selection marker is glutamine synthetase (GS) and a gene thereof. The GS-MSX system is a system effective in host cells lacking the GS gene (e.g., GS knockout CHO cells).

When the selective agent is an antibiotic, a degrading enzyme of the antibiotic, and its gene, an antibiotic resistance gene, are selection markers. Examples thereof include a hygromycin resistance gene, a neomycin resistance gene, a puromycin resistance gene, a chloramphenicol resistance gene, a tetracycline resistance gene, an erythromycin resistance gene, a spectinomycin resistance gene, a kanamycin resistance gene, a G418 resistance gene, a blasticidin resistance gene, a Zeocin resistance gene, a phleomycin resistance gene, and an ampicillin resistance gene.

In one example of embodiments of the vector set, the first expression vector contains the expression cassette of the first selection marker, the second expression vector contains the expression cassette of the second selection marker, and the first selection marker and the second selection marker are selection markers different from each other. The first selection marker is, for example, at least one selected from the group consisting of a dihydrofolate reductase gene, a glutamine synthetase gene, and an antibiotic resistance gene. The second selection marker is, for example, at least one selected from the group consisting of a dihydrofolate reductase gene, a glutamine synthetase gene, and an antibiotic resistance gene.

In one example of embodiments of the vector set, one of the first expression vector and the second expression vector contains the expression cassette of the dihydrofolate reductase gene or the glutamine synthetase gene, and the other contains the expression cassette of the antibiotic resistance gene. One preferred example of expression cassettes of the dihydrofolate reductase gene includes an mPGK promoter. One preferred example of expression cassettes of the glutamine synthetase gene includes an mPGK promoter. One preferred example of expression cassettes of the antibiotic resistance gene includes a virus-derived promoter.

One example of embodiments of the vector set for the bispecific antibody consisting of the first H-chain, the second H-chain, and the L-chain common to the first H-chain and the second H-chain includes a vector set below.

A vector set, in which the first expression vector contains the expression cassette of the L-chain, the expression cassette of the first H-chain, the expression cassette of the second H-chain, and the expression cassette of the first selection marker, and the second expression vector contains the expression cassette of the L-chain and the expression cassette of the second selection marker.

It is preferable that the vector set in the above form have the first selection marker and the second selection marker which are different from each other from the viewpoint of making the amount of subunit expressed from the first expression vector greater than the amount of subunit expressed from the second expression vector, and the first selection marker be a dihydrofolate reductase gene or a glutamine synthetase gene. In a more preferred form, the first selection marker is a dihydrofolate reductase gene or a glutamine synthetase gene, and the second selection marker is an antibiotic resistance gene. The expression vector containing a dihydrofolate reductase gene or a glutamine synthetase gene is more likely to increase in copy numbers within host cells than the expression vector containing an antibiotic resistance gene.

One example of embodiments of the vector set for the bispecific antibody consisting of the H-chain, the L-chain, and the scFv-Fc includes a vector set below.

A vector set, in which the first expression vector contains the expression cassette of the H-chain, the expression cassette of the L-chain, the expression cassette of the scFv-Fc, and the expression cassette of the first selection marker, and the second expression vector contains the expression cassette of the L-chain, the expression cassette of the scFv-Fc, and the expression cassette of the second selection marker.

A vector set, in which the first expression vector contains the expression cassette of the H-chain, the expression cassette of the L-chain, the expression cassette of the scFv-Fc, and the expression cassette of the first selection marker, and the second expression vector contains the expression cassette of the scFv-Fc, and the expression cassette of the second selection marker.

A vector set, in which the first expression vector contains the expression cassette of the H-chain, the expression cassette of the scFv-Fc, and the expression cassette of the first selection marker, and the second expression vector contains the expression cassette of the L-chain, the expression cassette of the scFv-Fc, and the expression cassette of the second selection marker.

A preferred aspect of the first selection marker and the second selection marker in the vector set in the above form is similar to the vector set for the bispecific antibody consisting of the first H-chain, the second H-chain, and the L-chain common to the first H-chain and the second H-chain.

Embodiments of the present disclosure do not exclude the use of a third expression vector other than the vector set (i.e., the first expression vector and the second expression vector). The third expression vector may be used for expressing polypeptides necessary for host cell growth or metabolism, for example.

One embodiment of the present disclosure is a mammalian cell that is transfected with the vector set. The mammalian cell transfected with the vector set may be a transiently expressing cell or a stably expressing cell of a heteromultimeric protein, and is preferably a stably expressing cell.

One embodiment of the present disclosure is a CHO cell that is transfected with the vector set. Specific examples thereof include CHO-DG44 cells, CHO-K1 cells, CHO-DXB11 cells, CHOpro3⁻ cells, which are transfected with the vector set, and established cell lines derived from these cells. The CHO cell transfected with the vector set may be a transiently expressing cell or a stably expressing cell of a heteromultimeric protein, and is preferably a stably expressing cells.

When the heteromultimeric protein is a bispecific antibody consisting of the first H-chain, the second H-chain, and the L-chain common to the first H-chain and the second H-chain, the mammalian cell and CHO cell transfected with the vector set are preferable such that the number of copies of the expression cassette of the L-chain be greater than a total of the number of copies of the expression cassette of the first H-chain and the number of copies of the expression cassette of the second H-chain. By this, the expression level and/or purity of the bispecific antibody increase.

The amount of mRNA measured by quantitative polymerase chain reaction (quantitative PCR) is useful to predict whether the number of copies of the expression cassettes present in the cells is large or small. In the mammalian cell and CHO cell transfected with the vector set for the bispecific antibody described above, the amount of mRNA measured by quantitative PCR is preferably such that, when the amount of mRNA of the first H-chain is 1, the amount of mRNA of the second H-chain be 0.5 to 2, and the amount of mRNA of the L-chain be 2 to 10. In the mammalian cell and CHO cell transfected with the vector set for the bispecific antibody described above, the amount of mRNA measured by quantitative PCR is preferably such that, when the amount of mRNA of the first H-chain is 1, the amount of mRNA of the second H-chain be 0.625 to 1.6, and the amount of mRNA of the L-chain be 3 to 8.

The mammalian cell and CHO cell transfected with the vector set are those expressing a heteromultimeric protein, and can be used for producing a heteromultimeric protein. The mammalian cell and CHO cell transfected with the vector set can also be used for administration, infusion or transplantation into a mammal.

An embodiment of the present disclosure is a method for producing a heteromultimeric protein. The method for producing a heteromultimeric protein includes culturing cells that produce a heteromultimeric protein. By culturing cells, heteromultimeric proteins are produced within cells, and accumulate in a culture solution and/or the cells.

Cells used in the method for producing a heteromultimeric protein are those produced by a method for producing cells, which is an embodiment of the present disclosure. These cells are selected according to evaluation criteria and/or relative production capacity of the heteromultimeric protein, and hence, they are excellent in productivity of heteromultimeric proteins.

The cell culturing method and medium composition for producing a heteromultimeric protein can be selected depending on the type of host cell. Culturing conditions (e.g., culture scaling, cell density, temperature and CO₂ concentration) can also be selected depending on the type of host cell.

One example of embodiments of the method for producing a heteromultimeric protein includes collecting a heteromultimeric protein from a culture solution. Examples of the method for collecting a heteromultimeric protein from a culture solution include centrifugation, filtration, diafiltration, ion exchange chromatography, affinity chromatography, hydrophobic interaction chromatography, gel filtration chromatography, and high performance liquid chromatography (HPLC). Collected heteromultimeric proteins are used for producing, for example, a pharmaceutical composition.

One example of embodiments of the method for producing a heteromultimeric protein includes collecting cells in which heteromultimeric proteins have accumulated from a medium. Examples of methods for collecting cells from a medium include centrifugation and filtration. Heteromultimeric proteins accumulate inside or on the surface of cells, depending on their nature. Collected cells are, for example, administered, infused, or transplanted into a mammal.

An embodiment of the present disclosure is a method for producing a cell pool. The method for producing a cell pool includes introducing a vector set into a host cell. The produced cell pool is subjected to selection of cells that produce a heteromultimeric protein. In the present disclosure, the cell pool refers to a cell population that has diversity in number of copies of expression cassettes of each subunit.

Examples of the scale of the cell pool include, but is not limited to, 10 to 10,000 types of clones.

The host cells for use in the method for producing a cell pool are preferably eukaryotic cells, more preferably mammalian cells, and further preferably CHO cells. Examples of the CHO cells include CHO-DG44 cells, CHO-K1 cells, CHO-DXB11 cells, CHOpro3⁻ cells, and established cell lines derived from these cells.

The cells contained in the cell pool may be transiently expressing cells or stably expressing cells of a heteromultimeric protein, and are preferably stably expressing cells.

The cell pool may be maintained by culturing cells, or may be stored by freezing.

### Examples

The following specific examples are given to describe a method for producing cells that produce a heteromultimeric protein, and a vector set in more detail. Materials, processing procedures, and the like, shown in the following specific examples can be appropriately changed without departing from the scope of the present disclosure. The ranges of the vector set of the present disclosure or the like should not be construed as being limited by the following specific examples.

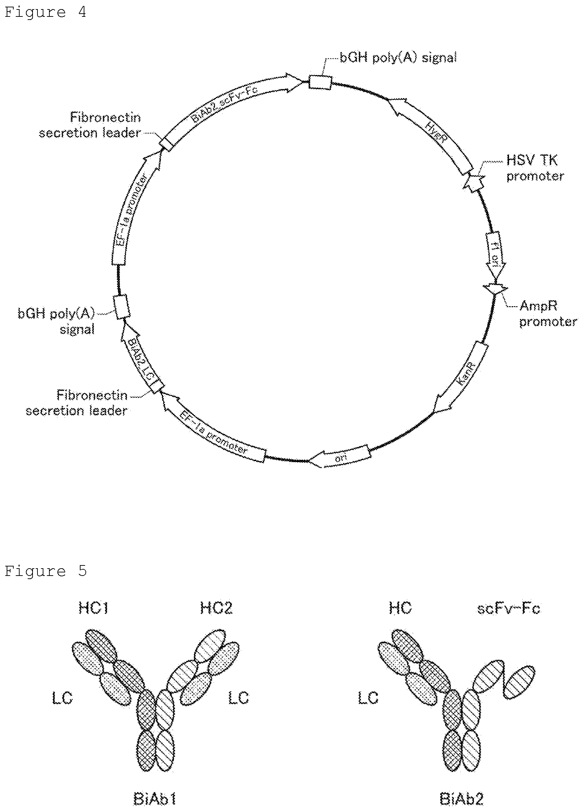

Note that configurations of the bispecific antibodies (BiAbl and BiAb2) produced in Examples 1 and 2 are shown in Figure 5.

### <<Example 1>>

### <Construction of Expression Vector>

Expression vectors 1 and 2 to express a tetrameric bispecific antibody consisting of two H-chains each having an amino acid sequence different from each other, and one L-chain common to the two H-chains were constructed.

Hereinafter, the above bispecific antibody is referred to as "BiAbl", the two H-chains are respectively referred to as "HC1" and "HC2", and the one L-chain is referred to as "common LC". HC1, HC2 and common LC are collectively referred to as "antibody subunit".

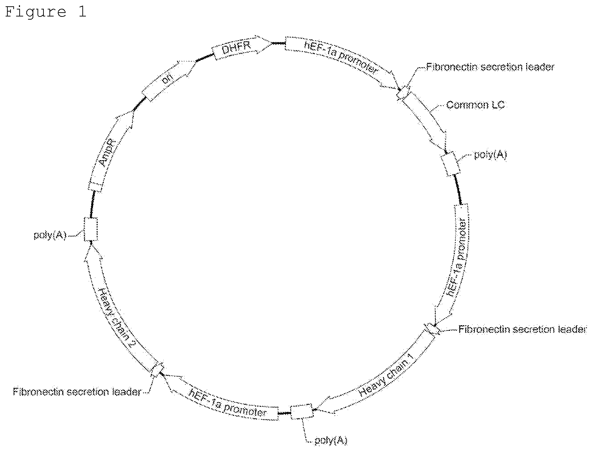

Figure 1 is a vector map of the expression vector 1. The expression vector 1 is a vector that contains one expression cassette of HC1, one expression cassette of HC2, one expression cassette of common LC, and one expression cassette of a DHFR gene. The expression cassettes of the antibody subunit each contain a hEF-1α promoter, a coding sequence of a fibronectin secretion leader, a coding sequence of the antibody subunit, and a polyA sequence; and the coding sequence of the antibody subunit are sequentially arranged downstream of the coding sequences of the fibronectin secretion leaders in the same reading frame.

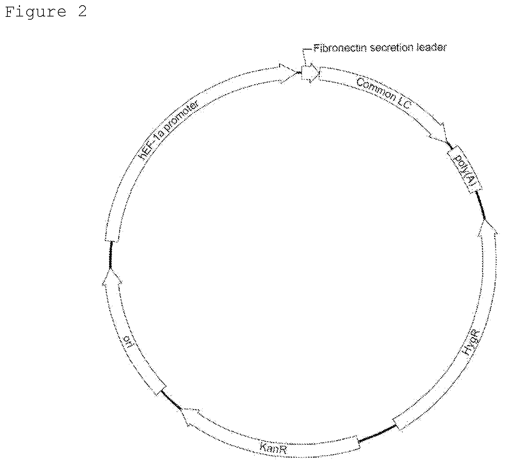

Figure 2 is a vector map of the expression vector 2. The expression vector 2 is a vector that contains one expression cassette of common LC, and one expression cassette of a hygromycin resistance gene. The expression cassette of common LC contains a hEF-1α promoter, a coding sequence of a fibronectin secretion leader, a coding sequence of common LC, and a polyA sequence; and the coding sequence of common LC is sequentially arranged downstream of the coding sequence of the fibronectin secretion leader in the same reading frame.

### <Introduction of Expression Vector into CHO Cells>

The expression vector 1 alone, or the expression vectors 1 and 2 were introduced into CHO-DG44 cells by an electroporation method.

The CHO-DG44 cells into which the expression vectors had been introduced were seeded in a 10 mL OptiCHO medium (Lifetechnologies, 12681011), and stationary cultured in an atmosphere at a temperature of 37°C and 5% CO₂ (v/v).

One day after the introduction of the expression vectors, the CHO-DG44 cells into which the expression vector 1 alone had been introduced were added with methotrexate as the selective agent, and the CHO-DG44 cells into which the expression vectors 1 and 2 had been introduced were concurrently added with methotrexate and hygromycin B as the selective agents.

14 days after the introduction of the expression vectors, the culture volume was expanded to 20 mL, the mixture was transferred to a 125 mL shaking culture flask for shake-culturing.

### <Selection of BiAb1 Producing Cells>

The cells that had been shake-cultured were dispensed at one cell per well in a 96-well plate using a droplet cell sorter, and stationary cultured in an atmosphere at a temperature of 37°C and 10% CO₂ (v/v). After culturing for 14 days, the culture supernatant was recovered, and the antibody concentrations were measured using molecular interaction analysis instrument Octet Qke (Sartorius). Clones with higher antibody concentrations were selected and cultured in a 24-well plate, and then cultured using a bioreactor tube for scaling up. As such, cells for producing BiAb1 were produced.

The BiAb1 producing cells produced by the introduction of the expression vector 1 alone are referred to as BiAb1-TGV cells. The BiAb1 producing cells produced by introducing the expression vectors 1 and 2 are referred to as BiAbl-TGV/SGV cells.

### <Production of BiAb1>

The BiAb1-TGV cells and BiAbl-TGV/SGV cells were each suspended in a 40 mL basal medium, transferred to a 125 mL shaking culture flask, and then, shake-cultured and fed at a rate of 140 rpm in an atmosphere at a temperature of 37°C and 5% CO₂ (v/v). A certain amount of feeding medium was added every day from the 3rd day from the culture start to the 13th day. Sampling was performed every one to three days, and the cell density, culture solution components and antibody concentrations were measured. On the 14th day from the culture start, the culture solution was recovered, and cells and cell debris were removed using a depth filter (pore size of 0.22 µm) to obtain a culture supernatant.

The antibody concentrations of the culture supernatants were measured by liquid chromatography using a protein A column. The antibodies were purified from the culture supernatant using a protein A column, and the quantification of BiAb1 and mispaired antibodies (antibodies in which only HCl is present as H-chain, and antibodies in which only HC2 is present as the H-chain) was performed using an icIEF analysis instrument Maurice (Protein Simple) to determine the purity of BiAb1. Here, the purity is determined by BiAb1/(BiAb1 + mispaired antibodies) × 100(%). Table 1 shows the measurement results on the clones in which antibody concentrations were the highest.

The amount of mRNA of the antibody subunit on each clone was measured by quantitative PCR. Table 2 shows the measurement results on the clones in which antibody concentrations were the highest. The numerical values shown in Table 2 are relative values when the amount of mRNA of HC1 in BiAb1-TGV cells is set to 1.

### [Table 1]

**Table 1**

| Clone | Titer [g/L] | Desired BiAb [%] | Desired BiAb [g/L] |
|---|---|---|---|
| BiAb1-TGV | 3.3 | 79.4 | 2.6 |
| BiAb1-TGV/SGV | 5.6 | 84.2 | 4.7 |

| [0143] | | | |
|---|---|---|---|
| [Table 2] | | | |
| Table 2 | | | |
| Clone | HC1 expression | HC2 expression | common LC expression |
| BiAb1-TGV | 1 | 0.8 | 2.1 |
| BiAb1-TGV/SGV | 1.1 | 0.7 | 6.2 |

As shown in Table 1, the purity of BiAb1 exceeded 75% (three quarters) in both cells. One expression cassette of HC1 and one expression cassette of HC2 are contained in the expression vector 1, and therefore, it is conceivable that BiAb1-TGV cells and BiAb1-TGV/SGV cells carry the expression cassette of HC1 and the expression cassette of HC2 at a number ratio of 1:1. In BiAb1-TGV cells and BiAbl-TGV/SGV cells, the expression level of HC1 and the Expression level of HC2 are comparable, and thus, it is presumed that mispaired antibodies are unlikely to occur.

The amount of BiAb1 was 2.6 g/L in BiAb1-TGV cells, and 4.7 g/L in BiAb1-TGV/SGV cells, which reveals that the amount in BiAbl-TGV/SGV cells is 1.8 times greater than in BiAb1-TGV cells.

As shown in Table 2, in BiAb1-TGV cells, the amount of mRNA of common LC was 2.1 times greater than the amount of mRNA of HC1 and 2.6 times greater than the amount of mRNA of HC2.

One expression cassette of HC1, one expression cassette of HC2, and one expression cassette of common LC are contained in the expression vector 1, and therefore, it is conceivable that BiAb1-TGV cells carry the expression cassette of HC1, the expression cassette of HC2, and the expression cassette of common LC at a number ratio of 1:1:1. It is presumed that the difference of the amounts of mRNA between the subunits is due to the difference of transcription efficiency of each expression cassette.

In BiAbl-TGV/SGV cells, the amount of mRNA of common LC was 5.6 times greater than the amount of mRNA of HC1 and 8.9 times greater than the amount of mRNA of HC2. It is conceivable that BiAbl-TGV/SGV cells carry the expression cassette of common LC more than the expression cassette of HC1 and the expression cassette of HC2. It is considered that this is the results of the introduction of both the expression vector 1 and the expression vector 2 into the host cells.

In BiAbl-TGV/SGV cells, the amount of mRNA of common LC was 3.4 times greater than the total amount of mRNA of HC1 and HC2. When simply predicting, the expression level of common LC is sufficient to be equal to a total of expression levels of HC1 and HC2, and therefore, the expression level of common LC in BiAbl-TGV/SGV cell can be said to be in excess (three times greater or more than the theoretically required amount). It is presumed that the fact that the expression level of common LC is in excess contributed to the increase in amount of BiAb1 in BiAb1-TGV/SGV cells (1.8 times greater than in BiAb1-TGV cells).

CHO cells in which the expression level and purity of BiAb1 were high were selected from CHO cells transfected with the vector set of the expression vector 1 and the expression vector 2, and therefore, CHO cells excellent in productivity of BiAb1 were able to be produced.

### <<Example 2>>

### <Construction of Expression Vector>

Expression vectors 3 and 4 to express a trimeric bispecific antibody consisting of one H-chain, one L-chain that paired with the H-chain, and one single-stranded antibody scFv-Fc were constructed.

Hereinafter, the above bispecific antibody is referred to as "BiAb2", the one H-chain is referred to as "HC", the one L-chain is referred to as " LC", and the one single-stranded antibody is referred to as "scFv-Fc". HC, LC and scFv-Fc are collectively referred to as "antibody subunit".

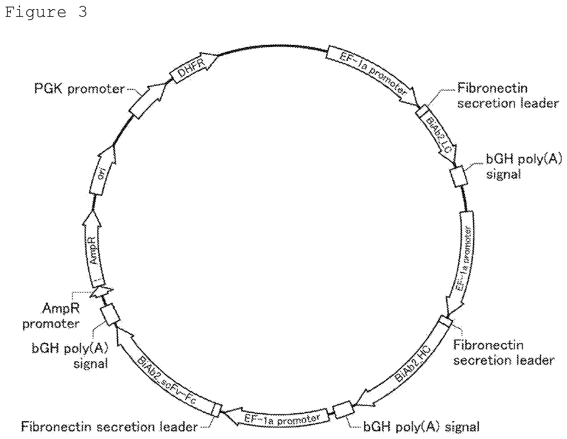

Figure 3 is a vector map of the expression vector 3. The expression vector 3 is a vector that contains one expression cassette of HC1, one expression cassette of LC, one expression cassette of scFv-Fc, and one expression cassette of a DHFR gene. The expression cassettes of the antibody subunit each contain a hEF-1α promoter, a coding sequence of a fibronectin secretion leader, a coding sequence of the antibody subunit, and a polyA sequence; and the coding sequences of the antibody subunit are sequentially arranged downstream of the coding sequences of the fibronectin secretion leaders in the same reading frame.

Figure 4 is a vector map of the expression vector 4. The expression vector 4 is a vector that contains one expression cassette of LC, one expression cassette of scFv-Fc, and one expression cassette of a hygromycin resistance gene. The expression cassette of LC contains a hEF-1α promoter, a coding sequence of a fibronectin secretion leader, a coding sequence of LC, and a polyA sequence; and the coding sequence of LC is sequentially arranged downstream of the coding sequence of the fibronectin secretion leader in the same reading frame. The expression cassette of scFv-Fc contains a hEF-1α promoter, a coding sequences of a fibronectin secretion leader, a coding sequence of scFv-Fc, and a polyA sequence; and the coding sequence of scFv-Fc is sequentially arranged downstream of the coding sequence of the fibronectin secretion leader in the same reading frame.

### <Introduction of Expression Vector into CHO Cells>

The expression vector 3 alone, or the expression vectors 3 and 4 were introduced into CHO-DG44 cells by an electroporation method.

The CHO-DG 44 cells into which the expression vectors had been introduced were seeded in a 10 mL OptiCHO medium (Lifetechnologies, 12681011), and stationary cultured in an atmosphere at a temperature of 37°C and 5% CO₂ (v/v).

One day after the introduction of the expression vectors, the CHO-DG44 cells into which the expression vectors 3 and 4 had been introduced were concurrently added with methotrexate and hygromycin B as the selective agents.

14 days after the introduction of the expression vectors, the culture volume was expanded to 20 mL, the mixture was transferred to a 125 mL shaking culture flask for shake-culturing.

The BiAb2 producing cells produced by the introduction of the expression vector 3 alone are referred to as BiAb2-TGV cells. The BiAb2 producing cells produced by introducing the expression vectors 3 and 4 are referred to as BiAb2-TGV/DGV cells.

### <Production of BiAb2>

The BiAb2-TGV cells and BiAb2-TGV/DGV cells were each suspended in a 40 mL basal medium, transferred to a 125 mL shaking culture flask, and then, shake-cultured and fed at a rate of 140 rpm in an atmosphere at a temperature of 37°C and 5% CO₂ (v/v). A certain amount of feeding medium was added every day from the 3rd day from the culture start to the 13th day. Sampling was performed every one to three days, and the cell density, culture solution components and antibody concentrations were measured. On the 14th day from the culture start, the culture solution was recovered, and cells and cell debris were removed using a depth filter (pore size of 0.22 µm) to obtain a culture supernatant.

The antibody concentrations in the culture supernatants were measured by liquid chromatography using a protein A column. The antibodies were purified from the culture supernatant using a protein A column, and the quantification of BiAb2 and mispaired antibodies (antibodies in which HC and scFv-Fc are not paired) was performed using a capillary electrophoresis analysis instrument PA800 plus (AB SCIEX) to determine the purity of BiAb2. Here, the purity is determined by BiAb2/ (BiAb2 + mispaired antibodies) × 100(%). Table 3 shows the productivity of each cell.

**[Table 3]**

| Clone | Titer [g/L] | Desired BiAb [%] | Desired BiAb [g/L] |
|---|---|---|---|
| BiAb2-TGV | 2.0 | 51 | 1.0 |
| BiAb2-TGV/DGV | 1.8 | 95 | 1.7 |

The amount of mRNA of the antibody subunit on each BiAb2 producing cell was measured by quantitative PCR. Table 4 shows the measurement results of each cell. The numerical values shown in Table 4 are relative values when the amount of mRNA of HC gene in BiAb2-TGV cells is set to 1.

**[Table 4]**

| Clone | HC expression | LC expression | scFV-Fc expression |
|---|---|---|---|
| BiAb2-TGV | 1.0 | 2.1 | 0.2 |
| BiAb2-TGV/DGV | 0.7 | 2.8 | 0.4 |

As shown in Table 3, the purity of BiAb2 in BiAb2-TGV cells was 51%, whereas the purity in BiAb2-TGV/DGV cells was 95%, significantly improving the purity. One expression cassette of HC and one expression cassette of scFv-Fc are contained in the expression vector 3, and therefore, it is conceivable that BiAb2-TGV cells and BiAb2-TGV/DGV cells carry the expression cassette of HC and the expression cassette of scFv-Fc at a number ratio of 1:1. However, the purity of BiAb2-TGV/DGV cells was significantly improved compared to BiAb2-TGV cells, and therefore, it is presumed that the expression levels of HC and scFv-Fc per copy number are different, whereby the purity of BiAb2 to be generated is reduced in BiAb2-TGV cells.

In BiAb2-TGV cells, the concentration of BiAb of interest was 1.0 g/L, whereas, in BiAb2-TGV/DGV cells, the concentration of BiAb of interest was 1.7 g/L.

As shown in Table 4, in BiAb2-TGV cells, the amount of mRNA of LC was 2.1 times greater than the amount of mRNA of HC and the amount of mRNA of scFv-Fc was 0.2 times greater than the amount of mRNA of HC.

One expression cassette of HC, one expression cassette of LC, and one expression cassette of scFv-Fc are contained in the expression vector 3, and therefore, it is conceivable that BiAb2-TGV cells carry the expression cassette of HC, the expression cassette of LC, and the expression cassette of scFv-Fc at a number ratio of 1:1:1. It was presumed that the difference of the amounts of mRNA between the subunits is due to the difference of transcription efficiency of each expression cassette, and in particular, the transcription efficiency of scFv-Fc was found out to be significantly low compared to those of HC and LC.

In BiAb2-TGV/DGV cells, the amount of mRNA of LC was 4 times greater than the amount of mRNA of HC and the amount of mRNA of scFv-Fc was about 0.5 times greater than the amount of mRNA of HC. It is conceivable that BiAb2-TGV/DGV cells carry the expression cassettes of LC and scFv-Fc more than the expression cassette of HC. It is considered that this is the results of the introduction of both the expression vector 3 and the expression vector 4 into the host cells.

In BiAb2-TGV/DGV cells, although the expression level of HC is 0.7 times greater than that of BiAb2-TGV cells, the production amount of BiAb2 of interest becomes high. It is considered that the improvement in the expression level of scFv-Fc caused the amount of HC-LC homodimer to be reduced, and BiAb2 was formed in the correct pair.

From these results, the additional introduction of a vector whose transcription efficiency is relatively low and that contain genes in which the expression level is insufficient allowed the expression balance to be improved, and productivity and purity of BiAb to be enhanced.

CHO cells in which the expression level and purity of BiAb2 were high were selected from CHO cells transfected with the vector set of the expression vector 3 and the expression vector 4, and therefore, CHO cells excellent in productivity of BiAb2 were able to be produced.

The disclosure of Japanese Patent Application No. 2022-094577 filed on June 10, 2022, is incorporated herein by reference in its entirety. All documents, patent applications, and technical standards mentioned herein are incorporated herein by reference to the same extent as if each individual document, patent application, and technical standard was specifically and individually indicated to be incorporated by reference.

## Claims

1. A method for producing cells that produce a heteromultimeric protein consisting of n types of subunits (wherein, n is an integer of 2 or higher), comprising:
introducing a vector set below into a host cell; and
selecting cells that produce a heteromultimeric protein from the host cell into which the vector set has been introduced,
wherein
the vector set is a set of a first expression vector and a second expression vector,
an expression cassette of at least one subunit X that is a portion of the n types of subunits is contained in both the first expression vector and the second expression vector, and
expression cassettes of subunits Y remaining after the subunit X is removed from the n types of subunits are contained in one of the first expression vector and the second expression vector for each type.

2. The method for producing cells according to claim 1, wherein the vector set is a vector set below,
wherein
the vector set is the set of the first expression vector and the second expression vector,
the expression cassette of at least one subunit X that is a portion of the n types of subunits is contained in both the first expression vector and the second expression vector, and
all of the expression cassettes of the subunits Y remaining after the subunit X is removed from the n types of subunits are contained in one of the first expression vector and the second expression vector.

3. The method for producing cells according to claim 1, wherein
the heteromultimeric protein consisting of the n types of subunits is a heteromultimeric protein consisting of two or more types and six or fewer types of subunits, and
the at least one subunit X is one type, two types, or three types of subunits X.

4. The method for producing cells according to claim 1, wherein
the heteromultimeric protein consisting of the n types of subunits is a heteromultimeric protein consisting of three or four types of subunits, and
the at least one subunit X is one type, two types, or three types of subunits X.

5. The method for producing cells according to claim 1, wherein
the heteromultimeric protein consisting of the n types of subunits is a heteromultimeric protein consisting of three types of subunits, and
the at least one subunit X is one type or two types of subunits X.

6. The method for producing cells according to claim 1, wherein
the heteromultimeric protein is an antibody.

7. The method for producing cells according to claim 1, wherein
the heteromultimeric protein is a bispecific antibody.

8. The method for producing cells according to claim 1, wherein
all of the expression cassettes of the subunits constituting the heteromultimeric protein comprise promoters of the same type.

9. The method for producing cells according to claim 8, wherein
the promoter is an hEF-1α promoter.

10. The method for producing cells according to claim 1, wherein
at least one of the expression cassettes of the subunits constituting the heteromultimeric protein comprises a coding sequence of a fibronectin secretion leader, and
in the expression cassettes comprising the coding sequence of the fibronectin secretion leader, a coding sequence of the subunits constituting the heteromultimeric protein is arranged downstream of the coding sequence of the fibronectin secretion leader in the same reading frame.

11. The method for producing cells according to claim 1, wherein
all of the expression cassettes of the subunits constituting the heteromultimeric protein comprise a coding sequence of a fibronectin secretion leader, and
a coding sequence of the subunits constituting the heteromultimeric protein is arranged downstream of the coding sequence of the fibronectin secretion leader in the same reading frame.

12. The method for producing cells according to claim 1, wherein
the first expression vector further comprises an expression cassette of a first selection marker, and
the second expression vector further comprises an expression cassette of a second selection marker.

13. The method for producing cells according to claim 12, wherein
the first selection marker and the second selection marker are selection markers different from each other,
the first selection marker is at least one selected from the group consisting of a dihydrofolate reductase gene, a glutamine synthetase gene, and an antibiotic resistance gene, and
the second selection marker is at least one selected from the group consisting of a dihydrofolate reductase gene, a glutamine synthetase gene, and an antibiotic resistance gene.

14. The method for producing cells according to claim 1, wherein
the host cell is a mammalian cell.

15. The method for producing cells according to claim 1, wherein
the host cell is a CHO cell.

16. The method for producing cells according to claim 1, wherein
the host cell is a CHO-DG44 cell, a CHO-K1 cell, a CHO-DXB11 cell, a CHOpro3⁻ cell, or an established cell line derived from these cells.

17. A method for producing cells that produce a bispecific antibody consisting of a first H-chain, a second H-chain, and an L-chain common to the first H-chain and the second H-chain, comprising:
introducing a vector set below into a host cell; and
selecting cells that produce a bispecific antibody from the host cell into which the vector set has been introduced,
wherein
the vector set is a set of the first expression vector and the second expression vector,
both the first expression vector and the second expression vector comprise an expression cassette of the L-chain,
one of the first expression vector and the second expression vector comprises an expression cassette of the first H-chain, and
one of the first expression vector and the second expression vector comprises an expression cassette of the second H-chain.

18. The method for producing cells according to claim 17, wherein
the first expression vector and the second expression vector each comprise one expression cassette of the L-chain,
one of the first expression vector and the second expression vector comprises one expression cassette of the first H-chain, and
one of the first expression vector and the second expression vector comprises one expression cassette of the second H-chain.

19. The method for producing cells according to claim 17, wherein
all of the expression cassettes of the subunits constituting the bispecific antibody comprise promoters of the same type.

20. The method for producing cells according to claim 19, wherein
the promoter is an hEF-1α promoter.

21. The method for producing cells according to claim 17, wherein
at least one of the expression cassettes of the subunits constituting the bispecific antibody comprises a coding sequence of a fibronectin secretion leader, and
in the expression cassettes comprising the coding sequence of the fibronectin secretion leader, a coding sequence of the subunits constituting the bispecific antibody is arranged downstream of the coding sequence of the fibronectin secretion leader in the same reading frame.

22. The method for producing cells according to claim 17, wherein
all of the expression cassettes of the subunits constituting the bispecific antibody comprise a coding sequence of a fibronectin secretion leader, and
a coding sequence of the subunits constituting the bispecific antibody is arranged downstream of the coding sequence of the fibronectin secretion leader in the same reading frame.

23. The method for producing cells according to claim 17, wherein
the first expression vector further comprises an expression cassette of a first selection marker, and
the second expression vector further comprises an expression cassette of a second selection marker.

24. The method for producing cells according to claim 23, wherein
the first expression vector comprises the expression cassette of the L-chain, the expression cassette of the first H-chain, the expression cassette of the second H-chain, and the expression cassette of the first selection marker, and
the second expression vector comprises the expression cassette of the L-chain and the expression cassette of the second selection marker.

25. The method for producing cells according to claim 24, wherein
the first selection marker and the second selection marker are selection markers different from each other, and
the first selection marker is a dihydrofolate reductase gene or a glutamine synthetase gene.

26. The method for producing cells according to claim 24, wherein
the first selection marker is a dihydrofolate reductase gene or a glutamine synthetase gene, and
the second selection marker is an antibiotic resistance gene.

27. The method for producing cells according to claim 17, wherein
the host cell is a mammalian cell.

28. The method for producing cells according to claim 17, wherein
the host cell is a CHO cell.

29. The method for producing cells according to claim 17, wherein
the host cell is a CHO-DG44 cell, a CHO-K1 cell, a CHO-DXB11 cell, a CHOpro3⁻ cell, or an established cell line derived from these cells.

30. A method for producing a heteromultimeric protein, comprising culturing cells that were produced by the method for producing cells according to any one of claims 1 to 16.

31. A method for producing a bispecific antibody, comprising culturing cells that were produced by the method for producing cells according to any one of claims 17 to 29.

32. A vector set that expresses a heteromultimeric protein consisting of n types of subunits (wherein, n is an integer of 2 or higher), wherein:
the vector set is a set of a first expression vector and a second expression vector,
an expression cassette of at least one subunit X that is a portion of the n types of subunits is contained in both the first expression vector and the second expression vector, and
expression cassettes of subunits Y remaining after the subunit X is removed from the n types of subunits are contained in one of the first expression vector and the second expression vector for each type.

33. The vector set according to claim 32, wherein
the vector set is the set of the first expression vector and the second expression vector,
the expression cassette of at least one subunit X that is a portion of the n types of subunits is contained in both the first expression vector and the second expression vector, and
all of the expression cassettes of the subunits Y remaining after the subunit X is removed from the n types of subunits are contained in one of the first expression vector and the second expression vector.

34. The vector set according to claim 32, wherein
the heteromultimeric protein consisting of the n types of subunits is a heteromultimeric protein consisting of two or more types and six or fewer types of subunits, and
the at least one subunit X is one type, two types, or three types of subunits X.

35. The vector set according to claim 32, wherein
the heteromultimeric protein consisting of the n types of subunits is a heteromultimeric protein consisting of three or four types of subunits, and
the at least one subunit X is one type, two types, or three types of subunits X.

36. The vector set according to claim 32, wherein
the heteromultimeric protein consisting of the n types of subunits is a heteromultimeric protein consisting of three types of subunits, and
the at least one subunit X is one type or two types of subunits X.

37. The vector set according to claim 32, wherein
the heteromultimeric protein is an antibody.

38. The vector set according to claim 32, wherein
the heteromultimeric protein is a bispecific antibody.

39. The vector set according to claim 32, wherein
all of the expression cassettes of the subunits constituting the heteromultimeric protein comprise promoters of the same type.

40. The vector set according to claim 39, wherein the promoter is an hEF-1α promoter.

41. The vector set according to claim 32, wherein
at least one of the expression cassettes of the subunits constituting the heteromultimeric protein comprises a coding sequence of a fibronectin secretion leader, and
in the expression cassettes comprising the coding sequence of the fibronectin secretion leader, a coding sequence of the subunits constituting the heteromultimeric protein is arranged downstream of the coding sequence of the fibronectin secretion leader in the same reading frame.

42. The vector set according to claim 32, wherein
all of the expression cassettes of the subunits constituting the heteromultimeric protein comprise a coding sequence of a fibronectin secretion leader, and
a coding sequence of the subunits constituting the heteromultimeric protein is arranged downstream of the coding sequence of the fibronectin secretion leader in the same reading frame.

43. The vector set according to claim 32, wherein
the first expression vector further comprises an expression cassette of a first selection marker, and
the second expression vector further comprises an expression cassette of a second selection marker.

44. The vector set according to claim 43, wherein
the first selection marker and the second selection marker are selection markers different from each other,
the first selection marker is at least one selected from the group consisting of a dihydrofolate reductase gene, a glutamine synthetase gene, and an antibiotic resistance gene, and
the second selection marker is at least one selected from the group consisting of a dihydrofolate reductase gene, a glutamine synthetase gene, and an antibiotic resistance gene.

45. A vector set that expresses a bispecific antibody consisting of a first H-chain, a second H-chain, and an L-chain common to the first H-chain and the second H-chain, wherein
the vector set is a set of a first expression vector and a second expression vector,
both the first expression vector and the second expression vector comprise an expression cassette of the L-chain,
one of the first expression vector and the second expression vector comprises an expression cassette of the first H-chain, and
one of the first expression vector and the second expression vector comprises an expression cassette of the second H-chain.

46. The vector set according to claim 45, wherein
the first expression vector and the second expression vector each comprise one expression cassette of the L-chain,
one of the first expression vector and the second expression vector comprises one expression cassette of the first H-chain, and
one of the first expression vector and the second expression vector comprises one expression cassette of the second H-chain.

47. The vector set according to claim 45, wherein
all of the expression cassettes of the subunits constituting the bispecific antibody comprise promoters of the same type.

48. The vector set according to claim 47, wherein
the promoter is an hEF-1α promoter.

49. The vector set according to claim 45, wherein
at least one of the expression cassettes of the subunits constituting the bispecific antibody comprises a coding sequence of a fibronectin secretion leader, and
in the expression cassettes comprising the coding sequence of the fibronectin secretion leader, a coding sequence of the subunits constituting the bispecific antibody is arranged downstream of the coding sequence of the fibronectin secretion leader in the same reading frame.

50. The vector set according to claim 45, wherein
all of the expression cassettes of the subunits constituting the bispecific antibody comprise a coding sequence of a fibronectin secretion leader, and
a coding sequence of the subunits constituting the bispecific antibody is arranged downstream of the coding sequence of the fibronectin secretion leader in the same reading frame.

51. The vector set according to claim 45, wherein
the first expression vector further comprises an expression cassette of a first selection marker, and
the second expression vector further comprises an expression cassette of a second selection marker.

52. The vector set according to claim 51, wherein
the first expression vector comprises the expression cassette of the L-chain, the expression cassette of the first H-chain, the expression cassette of the second H-chain, and the expression cassette of the first selection marker, and
the second expression vector comprises the expression cassette of the L-chain and the expression cassette of the second selection marker.

53. The vector set according to claim 52, wherein
the first selection marker and the second selection marker are selection markers different from each other,
the first selection marker is a dihydrofolate reductase gene or a glutamine synthetase gene.

54. The vector set according to claim 52, wherein
the first selection marker is a dihydrofolate reductase gene or a glutamine synthetase gene, and
the second selection marker is an antibiotic resistance gene.

55. A mammalian cell that is transfected with the vector set according to any one of claims 32 to 54.

56. A CHO cell that is transfected with the vector set according to any one of claims 32 to 54.

57. A mammalian cell that is transfected with the vector set according to any one of claims 45 to 54, wherein
when the amount of mRNA of the first H-chain is 1, the amount of mRNA of the second H-chain is 0.5 to 2, and the amount of mRNA of the L-chain is 2 to 10.

58. A mammalian cell that is transfected with the vector set according to any one of claims 45 to 54, wherein
when the amount of mRNA of the first H-chain is 1, the amount of mRNA of the second H-chain is 0.625 to 1.6, and the amount of mRNA of the L-chain is 3 to 8.

59. A CHO cell that is transfected with the vector set according to any one of claims 45 to 54, wherein
when the amount of mRNA of the first H-chain is 1, the amount of mRNA of the second H-chain is 0.5 to 2, and the amount of mRNA of the L-chain is 2 to 10.

60. A CHO cell that is transfected with the vector set according to any one of claims 45 to 54, wherein
when the amount of mRNA of the first H-chain is 1, the amount of mRNA of the second H-chain is 0.625 to 1.6, and the amount of mRNA of the L-chain is 3 to 8.

61. A method for producing a cell pool, comprising introducing the vector set according to any one of claims 32 to 54 into a host cell.

62. The method for producing a cell pool according to claim 61, wherein
the host cell is a mammalian cell.

63. The method for producing a cell pool according to claim 61, wherein
the host cell is a CHO cell.

64. The method for producing a cell pool according to claim 61, wherein
the host cell is a CHO-DG44 cell, a CHO-K1 cell, a CHO-DXB11 cell, a CHOpro3⁻ cell, or an established cell line derived from these cells.
